# EUROPEAN PATENT SPECIFICATION

(11) **EP 3 630 793 B1**
(45) Date of publication and mention of the grant of the patent: **10.07.2024**
(21) Application number: 18726972.5
(22) Date of filing: 23.05.2018
(51) Int. Cl.: C07K 7/08, C07K 14/33, C07K 14/315, C07K 17/00, C07K 1/16, C07K 14/31, C12N 15/62, C07K 1/22, C07K 16/00

(54) **A RECOMBINANT PROTEIN**
REKOMBINANTES PROTEIN
PROTÉINE RECOMBINANTE

(30) Priority: 24.05.2017 GB 201708277
(43) Date of publication of application: 08.04.2020
(60) Divisional application: 24178801.7
(73) Proprietor: Cytiva BioProcess R&D AB, 751 84 Uppsala (SE)
(72) Inventor: ANDER, Mats, 751 84 Uppsala (SE); BERGMAN, Magnus, 751 84 Uppsala (SE); BJORKMAN, Tomas, 751 84Uppsala (SE); GALLI, Joakim, 751 84 Uppsala (SE); RODRIGO, Gustav, 751 84 Uppsala (SE)
(74) Representative: Démoulin, Eva Lotta
(86) International application number: PCT/EP2018/063435
(87) International publication number: WO 2018/215503

(56) References cited:
- EP-A2- 2 532 672
- WO-A1-2012/083425
- WO-A1-2015/087848
- AJAMALUDDIN MALIK: "Protein fusion tags for efficient expression and purification of recombinant proteins in the periplasmic space of E. coli", 3 BIOTECH, vol. 6, no. 1, 4 February 2016 (2016-02-04), DE, XP055487226, ISSN: 2190-572X, DOI: 10.1007/s13205-016-0397-7
- GUAN K ET AL: "Eukaryotic proteins expressed in Escherichia coli: An improved thrombin cleavage and purification procedure of fusion proteins with glutathione S-transferase", ANALYTICAL BIOCHEMISTRY, ELSEVIER, AMSTERDAM, NL, vol. 192, no. 2, 1 February 1991 (1991-02-01), pages 262 - 267, XP024823753, ISSN: 0003-2697, [retrieved on 19910201], DOI: 10.1016/0003-2697(91)90534-Z
- S. W POLYAK ET AL: "Introduction of spacer peptides N-terminal to a cleavage recognition motif in recombinant fusion proteins can improve site-specific cleavage", PROTEIN ENGINEERING DESIGN AND SELECTION, 1 June 1997 (1997-06-01), pages 615 - 619, XP055272527, Retrieved from the Internet <URL:http://peds.oxfordjournals.org/content/10/6/615.full.pdf> [retrieved on 20160513], DOI: 10.1093/protein/10.6.615
- DATABASE Geneseq [online] 17 July 2014 (2014-07-17), "Escherichia coli polypeptide, SEQ ID 4290.", XP002782638, retrieved from EBI accession no. GSP:BBG83940 Database accession no. BBG83940
- DATABASE Geneseq [online] 13 August 2015 (2015-08-13), "Escherichia coli DsbA protein, SEQ ID 1.", retrieved from EBI accession no. GSP:BCB18497 Database accession no. BCB18497

## Description

### Technical field of the invention

The present invention relates to recombinant proteins, and more particularly to recombinant proteins expressed in gram-negative bacteria such as *Escherichia coli* (*E. coli*)*.* The invention also relates to nucleic acids, vectors and gram-negative bacteria for expression of recombinant proteins as well as to separation matrices with covalently linked recombinant protein ligands and to methods of separating immunoglobulins on such matrices.

### Background of the invention

Expression of heterologous proteins in *E. coli* (*E. coli*) is commonly used for recombinant proteins in laboratory and commercial scale. Expression in *E. coli* using secretion generally means transport of the produced protein over the inner membrane separating the cytoplasm and the periplasm. By secretion to the periplasm, the protein many times also leaks out to the extracellular medium (Mergulhão et al., Biotech Adv 23, 177-202, 2005). Secretion has many advantages compared to cytoplasmic expression such as facilitating correct protein folding, correct N-terminal processing, simplification of down-stream processing and prevention of aggregation into inclusion bodies. However far from all proteins have been successfully expressed in soluble form in periplasm. Some of the problems that might arise is poor secretion and no, or incorrect, processing of the signal peptide. A particular example of proteins having issues with inadequate secretion is in the expression of immunoglobulin binders based on the native or mutated Fc-binding domains B or C of *Staphylococcus aureus* Protein A (L Abrahmsén et al. EMBO J 4(13B), 3901-3906, 1985). Such immunoglobulin binders are commonly used as ligands in affinity chromatography separation of monoclonal antibodies, a major category of modern pharmaceuticals.

Protein expression is depending on a promoter sequence that starts the transcription of the messenger ribonucleotide acid (mRNA) followed by a ribosome-binding site (RBS) that attracts the translation machinery followed by a signal peptide sequence that is facilitating the transport of the protein to the periplasm. The mature protein is often cloned after the signal peptide and the mature protein is cleaved off from the signal peptide by a signal peptidase when passing the membrane. However, an issue when cloning constructs after a signal peptide is that the restriction enzymes often needs a specific sequence to cleave the DNA, and this leaves a cloning scar after the signal peptide sequence.

Accordingly, there is a need for improvements in the expression of heterologous proteins in A. *coli* and other gram-negative bacteria, in particular for immunoglobulin-binding proteins derived from the B and C domains of *Staphylococcus aureus* Protein A.

### Summary of the invention

One aspect of the invention is to provide a functional protein which is easily expressed and secreted in gram-negative bacteria like *E. coli.* This is achieved with a recombinant protein comprising an Fc-binding polypeptide and, linked to the N-terminus of said Fc-binding polypeptide, an N-terminal spacer having a length such that the distance between a signal peptide cleaving site and an N-terminus proximal structural unit of the Fc-binding polypeptide is 14-24 amino acid residues.

One advantage is that the expression level is improved by the introduction of the N-terminal spacer. A further advantage is that the selectivity of the signal peptide cleavage is improved.

A second aspect of the invention is to provide a nucleic acid molecule encoding for the recombinant protein. This is achieved with a nucleic acid molecule comprising the following elements in the 5' to 3' direction, said elements being operatively linked:
a) an inducible or constitutive promoter DNA sequence;
b) a DNA sequence encoding a signal peptide;
c) a DNA sequence encoding an N-terminal spacer; and
d) a DNA sequence encoding an immunoglobulin-binding polypeptide.

A third aspect of the invention is to provide a cloning vector which expresses and secretes the recombinant protein of any preceding claim into the bacterial periplasm of a gram-negative cell. This is achieved with a cloning vector comprising the above nucleic acid molecule.

A fourth aspect of the invention is to provide a gram-negative bacterium comprising the cloning vector.

A fifth aspect of the invention is to provide a method of expressing and secreting the recombinant protein in a gram-negative bacterium. This is achieved by a method comprising the steps of providing the gram-negative bacterium and culturing the gram-negative bacterium.

A sixth aspect of the invention is to provide a separation matrix comprising the recombinant protein covalently linked to a support.

A seventh aspect of the invention is to provide a method of separating an immunoglobulin, comprising the steps of:
i) providing the above separation matrix wherein the recombinant protein comprises an immunoglobulin-binding polypeptide;
ii) contacting the separation matrix with a liquid sample containing an immunoglobulin, to bind the immunoglobulin;
iii) optionally washing the separation matrix with a washing liquid;
iv) contacting the separation matrix with an elution liquid, to elute the immunoglobulin.

Further suitable embodiments of the invention are described in the dependent claims.

### Drawings

Fig. 1 shows an example sequence of cloning site where the spacer sequence was inserted. The restriction enzyme cleave sites are marked on top of the sequence.
Fig. 2 shows the integrated peak area at 237 nm from eluates of IgG Sepharose 6FF Tricorn 10 column.
Fig. 3 shows UV measurements at 210 nm, integrated peak area. a) pGE120 OmpA-AQGT (reference), 52% correct processed signal peptide. b) pGE144 OmpA-DsbA8AA, 96% correct signal peptide cleavage, c) pGE140 DsbA-DsbA8AA, 97% correct, signal peptide cleavage.
Fig. 4 shows the protein expression measured in heat treated fermentation broth for Zvar2₆, with and without the DsbA8AA N-terminal spacer, using concentration analysis with a standard curve. The arrow indicates the time of induction.
Fig. 5 shows UV measurements at 210 nm, integrated peak area. a pGE0002 OmpA-AQGT-Zvar2₆ (reference), b) pGE0180 OmpA-DsbA8AA- Zvar2₆.
Fig. 6 shows a schematic picture of the construct, with signal peptide, N-terminal spacer and Fc-binding polypeptide.
Fig. 7 shows a summary of the expression results from the shake flask cultivations. Error bars corresponding to one standard deviation are included where applicable. Three replicate measurements were made for construct pGE180. Two replicate measurements were made for samples DsbA7 and DsbA4. For all other constructs, one measurement was made.
Fig. 8 shows a) an example of the relevant total ion chromatogram (TIC) peak of a construct with correct signal peptide cleavage (pGE0180 in this case) and b) a construct with incorrect signal peptide cleavage (pGE0002 in this case).
Fig. 9 shows examples of deconvoluted TIC peaks after 24 hours of incubation in 1 M NaOH. The left image displays the typical pattern seen in pGE0180 (the cluster of peaks between 500 and 800 m/z). The right image displays the same area in DsbA8_noGT, where no peaks but the background are present.
Fig. 10 shows integrated extracted ion chromatogram (XIC) areas of peptide peaks corresponding to cleaved N-terminal sequences of different lengths after 0 hours (top left), 4 hours (top right) and 24 hours (bottom left). The areas are given in arbitrary units (AU).
Fig. 11 shows a zoomed-in view of the relevant peptide areas of the most promising candidates. The areas are given in arbitrary units (AU).
Fig. 12 shows a break-down of the most prominently appearing peptides in the constructs showing the lowest alkaline stability in the N-terminal region. The areas are given in arbitrary units (AU).
Fig. 13 shows the measured protein concentrations, protein concentrations estimated from extinction coefficients at 280 nm, and OD600 at the end of the fed-batch cultivations. One replicate run was made for pGE180, all other constructs were only cultivated once. Error bars corresponding to a 95% confidence interval are included where data is available.
Fig. 14 shows a comparison between the dynamic binding capacities of the candidate constructs (DsbA8_noGT and DsbA8_DT), the reference sample with Zvar2, and previous data from other immobilizations using base matrices with similar dry weights, porosities and ligand densities.

### Definitions

The terms "antibody" and "immunoglobulin" are used interchangeably herein, and are understood to include also fragments of antibodies, fusion proteins comprising antibodies or antibody fragments and conjugates comprising antibodies or antibody fragments.

The terms an "Fc-binding polypeptide" and "Fc-binding protein" mean a polypeptide or protein respectively, capable of binding to the crystallisable part (Fc) of an antibody and includes e.g. *Staphylococcus aureus* Protein A and *Streptococcus* Protein G, or any fragment or fusion protein thereof that has maintained said binding property.

The terms an "Fab-binding polypeptide" and "Fab-binding protein" mean a polypeptide or protein respectively, capable of binding to the antigen-binding part (Fab) of an antibody and includes e.g. *Peptostreptococcus magnus* Protein L, *Streptococcus* Protein G, native *Staphylococcus aureus* Protein A or any fragment or fusion protein thereof that has maintained said binding property.

The term "linker" herein means an element linking two polypeptide units, monomers or domains to each other in a multimer.

The term "% identity" with respect to comparisons of amino acid sequences is determined by standard alignment algorithms such as, for example, Basic Local Alignment Tool (BLAST^{™}) described in Altschul et al. (1990) J. Mol. Biol., 215: 403-410. A web-based software for this is freely available from the US National Library of Medicine at http://blast.ncbi.nlm.nih, gov/Blast.cgi?PROGRAM=blastp&PAGE_TYPE=BlastSearch&LINK _LOC=blasthome . Here, the algorithm "blastp (protein-protein BLAST)" is used for alignment of a query sequence with a subject sequence and determining i.a. the % identity.

The abbreviation "DsbA" herein means *E. coli,* Thiol:disulfide interchange protein, UniProt P0AEG4.

The abbreviation " OmpA" herein means *E. coli,* Outer membrane protein A, UniProt P0A910.

The abbreviation "PrA" herein means *Staphylococcus aureus* protein A, UniProt P38507.

The abbreviation "GIII" herein means Gene 3 from Bacteriophage M13, UniProt P69168.

The term "signal peptide" herein means a short (usually 16-30 amino acids long) peptide present at the N-terminus of the majority of newly synthesized proteins that are destined towards the secretory pathway. It may also be referred to as signal sequence, targeting signal, localization signal, localization sequence, transit peptide, leader sequence or leader peptide. The signal peptide is normally cleaved off from the protein by a signal peptidase enzyme.

The term "signal peptide cleavage site" herein means a dipeptide between which the signal peptidase cleaves the signal peptide from the mature protein. In most (but not all) cases the dipeptide is Ala-Ala. The signal peptide cleavage site can be calculated with algorithms such as SignalP 4.1, available on-line at http://www.cbs.dtu.dk/services/SignalP/
(Center for Biological Sequence Analysis, Technical University of Denmark).

The term "heterologous expression" herein means the expression of a gene or part of a gene in a host organism, which does not naturally have this gene or gene fragment. "Secreted" refers to across the inner membrane of gram negative bacteria, such as *E.coli.*

The cytoplasm (cytoplasmic), is the space inside of the inner cell membrane in gram negative bacteria containing the genetic material. The term "cytoplasmic expression" herein means protein expression within the cytoplasm.

The periplasm is a concentrated gel-like matrix in the space between the inner cytoplasmic membrane and the bacterial outer membrane called the periplasmic space in gram-negative bacteria.

The term "promoter" herein means a region of DNA that initiates transcription (writing to mRNA) of a particular gene. Promoters are normally located near the transcription start sites of genes, on the same strand and upstream on the DNA (towards the 5' region of the sense strand). A promoter can be inducible, meaning that the expression of genes operably linked to the promoter can be turned on by the presence of an inducer substance. Alternatively, the promoter may be constitutive, i.e. that it is not regulated by any inducer substance.

The abbreviation "RBS" herein means a ribosome-binding site, or ribosomal binding site. This is a sequence of nucleotides upstream of the start codon of an mRNA transcript that is responsible for the recruitment of a ribosome during the initiation of protein translation.

The abbreviation "RhaBAD" herein means the *E*. *coli* rhamnose operon promoter (also called rhamnose promoter) of genes RhaB, RhaA and RhaD. This is a promoter widely used in molecular biology.

The abbreviation "T5" herein means Bacteriophage T5 promoter for *E*. *coli* RNA polymerase, with an embedded lac operator. An operator is a segment of DNA to which a transcription factor binds to regulate gene expression by repressing it.

The abbreviation "pD861-SR" herein refers to a plasmid for *E*. *coli* protein expression with rhamnose promoter (RhaBAD) and a strong ribosome-binding site (SR).

The abbreviation "pJ401" herein refers to a plasmid for *E*. *coli* protein expression, with bacteriophage T5 promoter and dual embedded lac operator mirrored on each side of the promoter.

The abbreviation "OptEc" herein means optimized for *E.coli* expression, i.e. codon triplets are chosen to fit *E.coli* translation machinery.

The abbreviation "FspI" herein means a DNA restriction enzyme from Fischerella species (ATCC 29114). It cleaves blunt at the sequence TGCGCA.

The abbreviation "KpnI" herein means a DNA restriction enzyme from Klebsiella pneumoniae OK8 (ATCC 49790). It cleaves with overhang at the sequence GGTACC.

The abbreviation "SRP" herein means the signal recognition particle pathway, a universally conserved pathway for targeting polypeptides for secretion via the cotranslational pathway.

The abbreviation "Sec" herein means secretion or Type II secretory pathway, a system which is responsible for the secretion of proteins through the cell membrane.

The term "*E*. *coli* K12-017" herein means an *E. coli* expression strain, as described by Olsson M.O. and Isaksson L.A. in Molec. Gen. Genet. 169, 251-257 (1979).

The term "functional polypeptide" herein means a polypeptide with a technically useful property. Examples of such properties are: a) highly specific binding to a target species (for use as affinity binders, in particular as ligands for affinity chromatography), b) therapeutic properties (for use as a medicament), c) enzymatic properties (for use as biocatalysts) and d) signal-emitting properties (for use as reporter proteins, such as fluorescent reporter proteins).

As used herein, the terms "comprises," "comprising," "containing," "having" and the like can have the meaning ascribed to them in U.S. Patent law and can mean "includes," "including," and the like; "consisting essentially of or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### Detailed description of embodiments

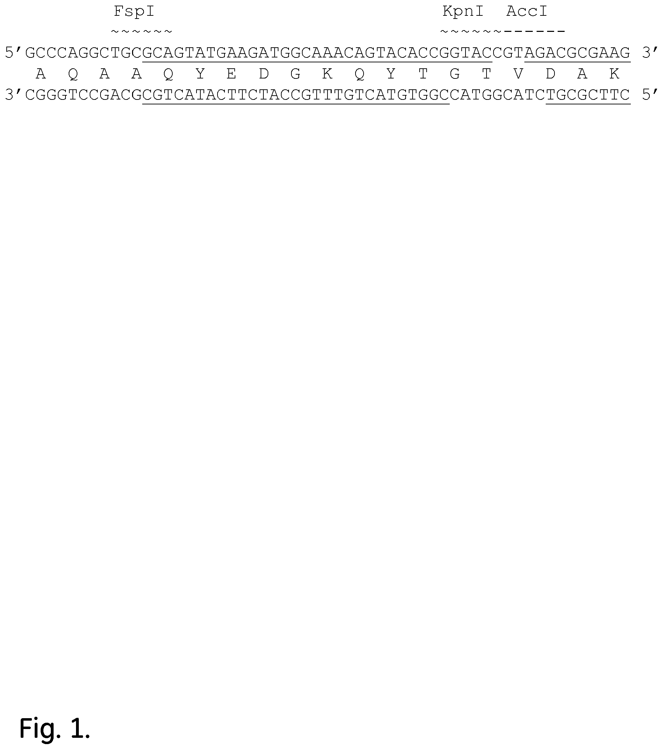

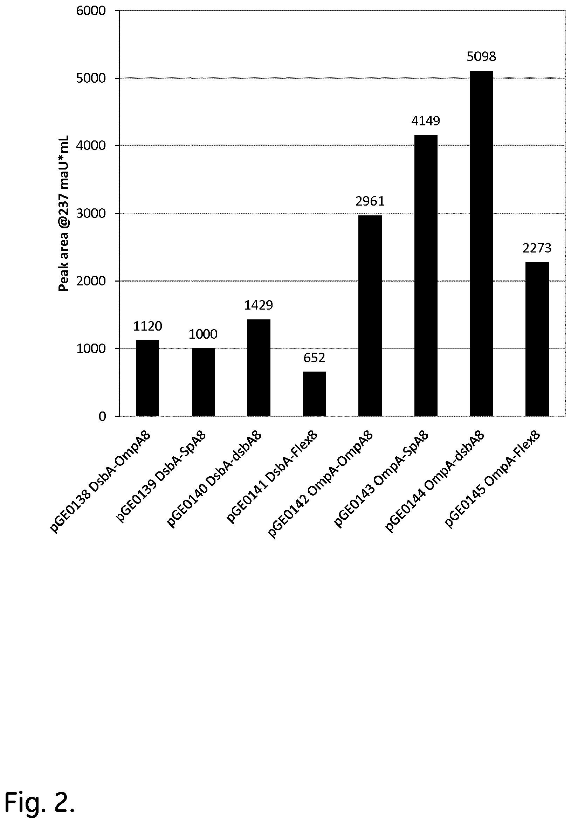

In one aspect, illustrated by Figs. 1-3, the present invention discloses a recombinant protein comprising a Fc-binding polypeptide and, linked to the N-terminus of the Fc-binding polypeptide, an N-terminal spacer having a length such that the number of amino acid residues between a signal peptide cleaving site and an N-terminus proximal structural unit of the Fc-binding polypeptide is 14-24.

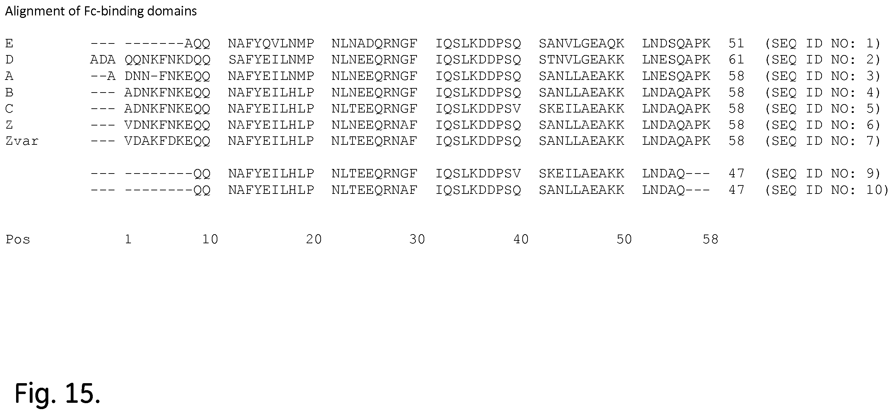

The Fc-binding polypeptide is an immunoglobulin-binding polypeptide. Such a polypeptide may e.g. comprise one or more immunoglobulin-binding domains derived from a bacterial protein selected from the group consisting of *Staphylococcus aureus* Protein A, *Peptostreptococcus magnus* Protein L and *Streptococcus* Protein G, such as from the group consisting of *Staphylococcus aureus* Protein A and *Peptostreptococcus magnus* Protein L. The immunoglobulin-binding domain(s) can e.g. have at least 80%, such as at least 90 or 95%, sequence identity with Domain E, D, A, B or C of *Staphylococcus aureus* Protein A, with Protein Z (a variant of Domain B of *Staphylococcus aureus* Protein A), Zvar or Zvar2 (alkali-stabilized mutants of Protein Z) or with Domain 1, 2, 3, 4 or 5 of *Peptostreptococcus magnus* Protein L. In this context, the immunoglobulin-containing domain(s) can be defined by, or have at least 80%, such as at least 90 or 95% sequence identity with, an amino acid sequence selected from the group consisting of SEQ ID NO: 1-11. SEQ ID NO. 1-7 (*Staphylococcus aureus* Protein A domains E, D, A, B and C, Protein Z and Zvar) are listed in Fig. 15, and SEQ ID NO. 8-11 are specified below.
SEQ ID NO. 8 - Zvar2 
VDAKFDKEAQ EAFYEILHLP NLTEEQRNAF IQSLKDDPSV SKAILAEAKK
LNDAQAPK
SEQ ID NO. 9 - truncated Domain C of *Staphylococcus aureus* Protein A
   QQ NAFYEILHLP NLTEEQRNGF IQSLKDDPSV SKEILAEAKK LNDAQ
SEQ ID NO.10 - truncated version of Zvar
   QQ NAFYEILHLP NLTEEQRNGF IQSLKDDPSV SKEILAEAKK LNDAQ
SEQ ID NO. 11 - truncated version of Zvar2
   AQ EAFYEILHLP NLTEEQRNAF IQSLKDDPSV SKAILAEAKK LNDAQ

SEQ ID NO. 1-11 can all be characterized as Fc-binding domains derived from *Staphylococcus aureus* Protein A. Such domains may further be alkali-stabilized by mutations of the native domains, as has been done in Zvar and Zvar2. Further examples of such alkali-stabilized domains can be SEQ ID NO. 48-93 (listed under Example 6) and other examples are given e.g. in US 8,329,860, US 8,754,196, US 9,040,661, US 9,403,883, JP 2006304633A, US 8,674,073, US 2010/0221844, US 2012/0208234, US 9,051,375, US 2014/0031522, US 2014/0107315, US 2013/0096276, US 2013/0274451, US 2005/0143566, US 2016/0159855, US 2016/0168209, US 2016/0237124, WO 2014/146350, WO 2016/079033, WO 2016/079034, WO 2016/152946, PCT EP2017/061162, PCT EP2017/061164, PCT EP2017/061160, PCT EP2017/061158, PCT EP2017/061159, US 14/961164, US 15/348699 and US 15/2823 67.

Specifically, the alkali-stabilized Fc-binding domains may have at least 80%, such as at least 90% or at least 95%, sequence identity to an amino acid sequence selected from the group consisting of SEQ ID NO. 7-11, 48-64 and 74-93.

The immunoglobulin-binding polypeptide can suitably be a multimer of immunoglobulin-binding domains, e.g. Fc-binding domains, as discussed above. The multimer can e.g. be a dimer, trimer, tetramer, pentamer, hexamer or a heptamer, such as a dimer, tetramer or hexamer. Suitably, the multimer may comprise at least four domains. The domains can be directly linked to each other (e.g. in the case of SEQ ID NO. 1-8, 48-64 and 74-93), but they can also be linked to each other via linkers, typically comprising 1-25 (such as 3-20) amino acid residues (e.g. in the case of SEQ ID NO 9-11). Examples of suitable linkers include APKADNKFNKE, APKVDAKFDKE, APK, APKVDA, AKFDKE, APKVFDKE, APAKFDKE, VDAKFDKE, APKKFDKE, APKYEDGKQYTVDAKFDKE and APKYEDGVDAKFDKE. Some specific examples of multimers include SEQ ID NO. 12 (tetramer), SEQ ID NO. 13 (hexamer) and SEQ ID NO. 65-73 (dimers).
SEQ ID NO 12 - Zvar tetramer with C-terminal cysteine 
VDAKFDKEQQ NAFYEILHLP NLTEEQRNAF IQSLKDDPSQ SANLLAEAKK
LNDAQAPK
VDAKFDKEQQ NAFYEILHLP NLTEEQRNAF IQSLKDDPSQ SANLLAEAKK
LNDAQAPK
VDAKFDKEQQ NAFYEILHLP NLTEEQRNAF IQSLKDDPSQ SANLLAEAKK
LNDAQAPK
VDAKFDKEQQ NAFYEILHLP NLTEEQRNAF IQSLKDDPSQ SANLLAEAKK
LNDAQAPKC
SEQ ID NO 13 - Zvar2 hexamer with C-terminal cysteine

VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV
SKAILAEAKK LNDAQAPK
VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV
SKAILAEAKK LNDAQAPK VDAKFDKEAQ EAFYEILHLP NLTEEQRNAF
IQSLKDDPSV SKAILAEAKK LNDAQAPK VDAKFDKEAQ EAFYEILHLP
NLTEEQRNAF IQSLKDDPSV SKAILAEAKK LNDAQAPKC

The recombinant protein may comprise a coupling moiety at or proximal to the C-terminus or the N-terminus, such as at the C-terminus. This coupling moiety can be used for specific coupling to a support as discussed below and may comprise a cysteine, allowing for coupling by thioether bonds. Alternatively, or additionally, the coupling moiety may comprise one or more lysines, such as a cluster of 2-8 lysines.

The Fc-binding polypeptide may have secondary and tertiary structures and may suitably comprise one or more structural units, as exemplified by alpha helices, beta sheets and/or beta barrels. In particular, the polypeptide may comprise a plurality of alpha helices, such as at least three alpha helices. The Fc-binding domains derived from *Staphylococcus aureus* Protein A (e.g. SEQ ID NO. 1-11, 48-64 and 74-93) each comprise three alpha helices, so the number of alpha helices in a multimer as discussed above may be three times the number of domains in the multimer. The first alpha helix in the domains as exemplified by SEQ ID NO. 1-11, 48-64 and 74-93 starts at position 9 (using the position nomenclature of Fig. 15), which in the case of SEQ ID NO 1-7 and 9-10 is a glutamine and in SEQ ID NO. 8, 11, 48-64 and 74-93 is an alanine.

The N-terminal spacer suitably has a length such that the number of amino acid residues between the signal peptide cleaving site and an N-terminus proximal structural unit of the Fc-binding polypeptide is 14-24. As discussed above, this structural unit can suitably be an alpha helix (or alternatively a beta sheet or beta barrel). The number of amino acid residues between the N-terminus of the Fc-binding polypeptide and the N-terminus proximal structural unit can vary, e.g. between 0 (SEQ ID NO. 9-11) and 11 (SEQ ID NO. 2), and accordingly, the length of the N-terminal spacer may vary, e.g. between 3-24 amino acid residues, such as between 8-24 amino acid residues or 14-24 amino acid residues. For example, if the N-terminal spacer is linked to an Fc-binding domain having at least 90% identity to SEQ ID NO. 1-8, 48-64 or 74-93, the N-terminal spacer may e.g. have a length of 8-12 amino acid residues and if the N-terminal spacer is linked to an Fc-binding domain having at least 90% identity to SEQ ID NO. 9-11, the N-terminal spacer may e.g. have a length of 16-20 amino acid residues. The N-terminal spacer may e.g. consist of amino acid residues selected from the group consisting of alanine, asparagine, aspartic acid, glutamine, glutamic acid, glycine, histidine, lysine, phenylalanine, serine, threonine, tryptophan, tyrosine and valine. For improved alkali stability, it may be advantageous to exclude asparagines. In this case, the N-terminal spacer may e.g. consist of amino acid residues selected from the group consisting of alanine, aspartic acid, glutamine, glutamic acid, glycine, histidine, lysine, phenylalanine, serine, threonine, tryptophan, tyrosine and valine. It can be advantageous if the N-terminal spacer does not comprise any clusters of arginines or lysines, and/or if it comprises at most two amino acid residues selected from the group consisting of lysine and arginine. The two N-terminal amino acid residues of the N-terminal spacer are AQ (alanine followed by glutamine).

In particular, the N-terminal spacer may comprise, comprise essentially or have, an amino acid sequence having at least 80% sequence identity to, or being defined by, an amino acid sequence selected from the group consisting of SEQ ID NO. 16-18, 29-30, 33-40, 43-45 and 47. The N-terminal spacer may further comprise, comprise essentially or have an amino acid sequence having at least 80% sequence identity to, or being defined by, an amino acid sequence selected from the group consisting of AQYEDGKQYTADNKFNKE, AQYEDGKQYTVDAKFDKE, AQKDQTWYTGVDAKFDKE, AQHDEAQQEAVDAKFDKE, AQGGGSGGGSVDAKFDKE, AQYEDGKQYGTVDAKFDKE, AQYEDGKQGTVDAKFDKE, AQYEDGKQYTTLEKGTVDAKFDKE, AQYEDGKQYTTLEKPVAGGTVDAKFDKE, AQYEDGKQYTVDAKFDKE, AQYEDGKQYTETVDAKFDKE, AQYEDGKQYTDTVDAKFDKE, AQYEDGKQYTATVDAKFDKE, AQYEDGKQYEDTVDAKFDKE, AQHHHHHHHHGTVDAKFDKE, AQHHHHHHGTVDAKFDKE and AQHDEAQQEAGTVDAKFDKE. The latter sequences are particularly advantageous in combination with Fc-bindingpolypeptides derived from SEQ ID NO. 9-11.

In terms of alkali stability it can further be advantageous to use an N-terminal spacer comprising, comprising essentially or having, an amino acid sequence having at least 80% sequence identity to, or being defined by, an amino acid sequence selected from the group consisting of SEQ ID NO. 35, 37, 38, 40, 43, 44 and 47. The N-terminal spacer may also for alkali stability comprise, comprise essentially or have an amino acid sequence having at least 80% sequence identity to, or being defined by, an amino acid sequence selected from the group consisting of AQYEDGKQYTVDAKFDKE, AQYEDGKQYTETVDAKFDKE, AQYEDGKQYTDTVDAKFDKE, AQYEDGKQYEDTVDAKFDKE, AQHHHHHHHHGTVDAKFDKE, AQHHHHHHGTVDAKFDKE and AQHDEAQQEAGTVDAKFDKE. The latter sequences are particularly advantageous in combination with immunoglobulin-binding polypeptides derived from SEQ ID NO. 9-11.

The alkali stability of the recombinant protein can be assessed by coupling it to an SPR chip, e.g. to Biacore CM5 sensor chip as described in the examples of WO2016079033, using e.g. NHS- or maleimide coupling chemistries, and measuring the immunoglobulin-binding capacity of the chip, typically using polyclonal human IgG, before and after incubation in alkaline solutions at a specified temperature, e.g. 22 +/- 2 °C. The incubation can e.g. be performed in 0.5 M NaOH for a number of 10 min cycles, such as 100, 200 or 300 cycles. The IgG capacity of the matrix after 100 10 min incubation cycles in 0.5 M NaOH at 22 +/- 2 °C can be at least 55, such as at least 60, at least 80 or at least 90% of the IgG capacity before the incubation. Alternatively, the remaining IgG capacity after 100 cycles for a particular mutant measured as above can be compared with the remaining IgG capacity for a parental recombinant protein. In this case, the remaining IgG capacity for the mutant may be at least 105%, such as at least 110%, at least 125%, at least 150% or at least 200% of the parental recombinant protein.

In a second aspect, the present invention discloses a nucleic acid molecule encoding for the recombinant protein as disclosed above. The nucleic acid molecule comprises the following operatively linked elements in the 5' to 3' direction:
a) an inducible or constitutive promoter DNA sequence, e.g. the RhaBAD or T5 promoter sequence. RhaBAD is inducible with rhamnose and T5 with Isopropyl β-D-1-thiogalactopyranoside (IPTG). An example of a constitutive promoter is the spa promoter (the promoter of the naturally occurring *Staphylococcus aureus* Protein A);
b) a DNA sequence encoding a signal peptide, e.g. the OmpA (SEQ ID NO. 14) or DsbA (SEQ ID NO. 15) signal peptides, or a signal peptide having at least 80% sequence identity with either of these;
c) a DNA sequence encoding an N-terminal spacer as discussed above; and
d) a DNA sequence encoding an Fc-binding polypeptide as discussed above.

The nucleic acid molecule may further comprise a ribosome-binding site (RBS) and an origin of replication. It can suitably also comprise an antibiotic resistance marker.

In a third aspect, the present invention discloses a cloning vector, e.g. a plasmid, which expresses and secretes the recombinant protein as disclosed above into the bacterial periplasm of a gram-negative cell, e.g. *E. coli.* The cloning vector comprises the nucleic acid molecule as discussed above.

In a fourth aspect, the present invention discloses a gram-negative bacterium comprising the cloning vector disclosed above. The bacterium may e.g. be identified as *Escherichia coli,* in particular *E. coli* of the K12 strain, such as *E. coli* K12-017. Other examples of gram-negative bacteria include e.g. the genus *Pseudomonas,* such as e.g. *Pseudomonas fluorescens.* The transformation may be achieved e.g. by the heat shock method, although other methods such as electroporation are also possible.

In a fifth aspect, the present invention discloses a method of expressing and secreting the recombinant protein discussed above in a gram-negative bacterium. This method comprises the steps of:
i) providing the gram-negative bacterium disclosed above; and
ii) culturing the gram-negative bacterium.

If the nucleic acid molecule as discussed above comprises an inducible promoter, the method may further comprise a step of inducing recombinant protein expression in the gram-negative bacterium.

In a sixth aspect, the present invention discloses a separation matrix comprising the recombinant protein disclosed above, covalently linked to a support.

As the skilled person will understand, the expressed recombinant protein should be purified to an appropriate extent before being immobilized to a support. Such purification methods are well known in the field, and the immobilization of protein-based ligands to supports is easily carried out using standard methods. Suitable methods and supports will be discussed below in more detail.

The alkali stability of the matrix can be assessed by measuring the immunoglobulin-binding capacity, typically using polyclonal human IgG, before and after incubation in alkaline solutions at a specified temperature, e.g. 22 +/- 2 °C. The incubation can e.g. be performed in 0.5 M NaOH for a number of 15 min cycles, such as 100, 200 or 300 cycles, corresponding to a total incubation time of 25, 50 or 75 h. The IgG capacity of the matrix after 96-100 15 min incubation cycles or a total incubation time of 24 or 25 h in 0.5 M NaOH at 22 +/- 2 °C can be at least 80, such as at least 85, at least 90 or at least 95% of the IgG capacity before the incubation.

The solid support of the matrix according to the invention can be of any suitable well-known kind. A conventional affinity separation matrix is often of organic nature and based on polymers that expose a hydrophilic surface to the aqueous media used, i.e. expose hydroxy (-OH), carboxy (-COOH), carboxamido (-CONH₂, possibly in N- substituted forms), amino (-NH₂, possibly in substituted form), oligo- or polyethylenoxy groups on their external and, if present, also on internal surfaces. The solid support can suitably be porous. The porosity can be expressed as a Kav or Kd value (the fraction of the pore volume available to a probe molecule of a particular size) measured by inverse size exclusion chromatography, e.g. according to the methods described in Gel Filtration Principles and Methods, Pharmacia LKB Biotechnology 1991, pp 6-13. By definition, both Kd and Kav values always lie within the range 0-1. The Kav value can advantageously be 0.6 - 0.95, e.g. 0.7 - 0.90 or 0.6 - 0.8, as measured with dextran of Mw 110 kDa as a probe molecule. An advantage of this is that the support has a large fraction of pores able to accommodate both the recombinant protein of the invention and immunoglobulins binding to the recombinant protein and to provide mass transport of the immunoglobulins to and from the binding sites.

The recombinant protein may be attached to the support via conventional coupling techniques utilising e.g. thiol, amino and/or carboxy groups present in the ligand. Bisepoxides, epichlorohydrin, CNBr, N-hydroxysuccinimide (NHS) etc are well-known coupling reagents. Between the support and the recombinant protein, a molecule known as a spacer can be introduced, which improves the availability of the recombinant protein and facilitates the chemical coupling of the recombinant protein to the support. Depending on the nature of the recombinant protein and the coupling conditions, the coupling may be a multipoint coupling (e.g. via a plurality of lysines) or a single point coupling (e.g. via a single cysteine). Alternatively, the recombinant protein may be attached to the support by non-covalent bonding, such as physical adsorption or biospecific adsorption.

In some embodiments, the matrix comprises 5 - 25, such as 5-20 mg/ml, 5 - 15 mg/ml, 5-11 mg/ml or 6 - 11 mg/ml of the recombinant protein coupled to the support. The amount of coupled protein can be controlled by the concentration of protein used in the coupling process, by the activation and coupling conditions used and/or by the pore structure of the support used. As a general rule, the absolute binding capacity of the matrix increases with the amount of coupled protein, at least up to a point where the pores become significantly constricted by the coupled protein. The relative binding capacity per mg coupled protein will decrease at high coupling levels, resulting in a cost-benefit optimum within the ranges specified above.

In certain embodiments, the protein is coupled to the support via thioether bonds. Methods for performing such coupling are well-known in this field and easily performed by the skilled person in this field using standard techniques and equipment. Thioether bonds are flexible and stable and generally suited for use in affinity chromatography. In particular, when the thioether bond is via a terminal or near-terminal cysteine residue on the recombinant protein, the mobility of the coupled recombinant protein is enhanced which provides improved binding capacity and binding kinetics. In some embodiments, the recombinant protein is coupled via a C-terminal cysteine provided on the protein as described above. This allows for efficient coupling of the cysteine thiol to electrophilic groups, e.g. epoxide groups, halohydrin groups etc. on a support, resulting in a thioether bridge coupling. Alternatively, the recombinant protein may be covalently linked to the support via one or more amide bonds. This can be achieved e.g. through reaction between one or more lysines in the protein and one or more activated carboxyl groups on the support. The activation can e.g. be made by the commonly known N-hydroxysuccinimide (NHS) reagent. Yet another alternative is that the protein is linked via one or more secondary amine links. Such links can be formed from lysines in the protein and either hydroxyl groups on the support, which have been activated using e.g. tresyl chloride or tosyl chloride chemistry, or by reductive amination reaction between the lysines and aldehydes on the support. The aldehydes can e.g. be formed from vicinal diols on the support through periodate oxidation.

In certain embodiments, the support comprises a polyhydroxy polymer, such as a polysaccharide. Examples of polysaccharides include e.g. dextran, starch, cellulose, pullulan, agar, agarose etc. Polysaccharides are inherently hydrophilic with low degrees of nonspecific interactions, they provide a high content of reactive (activatable) hydroxyl groups and they are generally stable towards alkaline cleaning solutions used in bioprocessing.

In some embodiments, the support comprises agar or agarose. The supports used in the present invention can easily be prepared according to standard methods, such as inverse suspension gelation (S Hjertén: Biochim Biophys Acta 79(2), 393-398 (1964). Alternatively, the base matrices are commercially available products, such as crosslinked agarose beads sold under the name of SEPHAROSE^{™} FF (GE Healthcare). In an embodiment, which is especially advantageous for large-scale separations, the support has been adapted to increase its rigidity using the methods described in US6602990 or US7396467, and hence renders the matrix more suitable for high flow rates.

In certain embodiments, the support, such as a polysaccharide or agarose support, is crosslinked, such as with hydroxyalkyl ether crosslinks. Crosslinker reagents producing such crosslinks can be e.g. epihalohydrins like epichlorohydrin, diepoxides like butanediol diglycidyl ether, allylating reagents like allyl halides or allyl glycidyl ether. Crosslinking is beneficial for the rigidity of the support and improves the chemical stability. Hydroxyalkyl ether crosslinks are alkali stable and do not cause significant nonspecific adsorption.

Alternatively, the solid support is based on synthetic polymers, such as polyvinyl alcohol, polyhydroxyalkyl acrylates, polyhydroxyalkyl methacrylates, polyacrylamides, polymethacrylamides etc. In case of hydrophobic polymers, such as matrices based on divinyl and monovinyl-substituted benzenes, the surface of the matrix is often hydrophilised to expose hydrophilic groups as defined above to a surrounding aqueous liquid. Such polymers are easily produced according to standard methods, see e.g. "Styrene based polymer supports developed by suspension polymerization" (R Arshady: Chimica e L'Industria 70(9), 70-75 (1988)). Alternatively, a commercially available product, such as SOURCE^{™} (GE Healthcare) is used. In another alternative, the solid support according to the invention comprises a support of inorganic nature, e.g. silica, zirconium oxide etc.

In certain embodiments, the solid support is in another form such as a surface, a chip, capillaries, or a filter (e.g. a membrane or a depth filter matrix).

In a seventh aspect, the present invention discloses a method of separating an immunoglobulin, comprising the steps of:
i) providing the separation matrix as discussed above, wherein the recombinant protein comprises an immunoglobulin-binding polypeptide, e.g. comprising one or more Fc-binding domains derived from *Staphylococcus aureus* Protein A;
ii) contacting the separation matrix with a liquid sample containing an immunoglobulin, to bind the immunoglobulin;
iii) optionally washing the separation matrix with a washing liquid;
iv) contacting the separation matrix with an elution liquid, to elute the immunoglobulin;
v) optionally cleaning the separation matrix with a cleaning liquid. Alkali cleaning liquids are commonly used in bioprocessing and, provided that the recombinant protein is alkali-stable, the cleaning liquid may comprise at least 0.1 M NaOH or KOH, such as at least 0.5 M NaOH or KOH, or 0.5-2.5 M NaOH or KOH.

### EXAMPLES

First, two different signal peptides were tested together with four different N-terminal spacers to find the best cleavage and protein expression. This experiment (Example 1) was performed with Zvar2 monomer using the RhaBAD promoter system. In Example 2, the most promising signal peptide and N-terminal start was cloned in to Zvar2 hexamer. This construct was tested in fed-batch fermentations with and without the N-terminal start sequence. This study was performed using the T5 promoter expression system. In Example 3, all the native domains of *Staphylococcus aureus* protein A, Zvar monomer (Zvar₁), Zvar2 monomer (Zvar2₁) and Zvar tetramer (Zvar₄) were tested with the chosen signal peptide and N-terminal start to see if the start sequence also had an effect on closely related domains. Also Example 3 was performed with the T5 expression system. Example 4 was performed with a set of different N-terminal spacers, Example 5 was a scale-up of fermentations using a couple of selected spacers and Example 6 was an investigation using the AQYEDGKQYTGT N-terminal spacer in combination with different mutants of an immunoglobulin-binding protein.

### Materials and methods

### Constructs

Genes as described in Table 1 were synthesized by a contract manufacturer of synthetic genes (ATUM, CA, USA). Double stranded dideoxy ribonucleic acid (dsDNA) was synthesized based on the amino acid (AA) sequence and was optimized for expression in *E. coli* by a manufacturer proprietary algorithm. The dsDNA was inserted into expression vector pD861-SR or pJ401 with a signal peptide either from DsbA or OmpA (Table 2).

**Table 1. Plasmids ordered from ATUM.**

| Plasmid number | Insert | Alias | Promoter | Signal peptide | N-terminal start | Cloning vector | Insert sequence |
|---|---|---|---|---|---|---|---|
| pGE0002 | Zvar2₆ | Zvar2₆ | T5 | OmpA | AQGT | pJ401 | (VDAKFDKEAQ EAFYEILHLP NLTEEQRNAF IQSLKDDPSV SKAILAEAKK LNDAQAPK)x6C |
| pGE0068 | Zvar2₁ | Zvar2₁ | RhaBAD | DsbA | AQGT | pD861-SR | VDAKFDKEAQ EAFYEILHLP NLTEEQRNAF IQSLKDDPSV SKAILAEAKK LNDAQAPKC |
| pGE0091 | Zvar₄_Opt Ec | Zvar₄ | T5 | OmpA | AQGT | pJ401 | (VDAKFDKEQQ NAFYEILHLP NLTEEQRNAF IQSLKDDPSQ SANLLAEAKK LNDAQAPK)x4C |
| pGE0096 | Zvar₁_Opt Ec | Zvar₁ | T5 | OmpA | AQGT | pJ401 | VDAKFDKEQQ NAFYEILHLP NLTEEQRNAF IQSLKDDPSQ SANLLAEAKK LNDAQAPKC |
| pGE0119 | Zvar2₁ | Zvar2₁ | RhaBAD | OmpA | AQGT | pD861-SR | VDAKFDKEAQ EAFYEILHLP NLTEEQRNAF IQSLKDDPSV SKAILAEAKK LNDAQAPKC |
| pGE0120 | Zvar2₁ | Zvar2₁ | T5 | OmpA | AQGT | pJ401 | VDAKFDKEAQ EAFYEILHLP NLTEEQRNAF IQSLKDDPSV SKAILAEAKK LNDAQAPKC |
| pGE0127 | E-domain | E-domain | T5 | OmpA | AQGT | pJ401 | *VDEAQQNAFY QVLNMPNLNA DQRNGFIQSL KDDPSQSANV LGEAQKLNDSQ APKC |
| pGE0128 | D-domain | D-domain | T5 | OmpA | AQGT | pJ401 | *VDAQQNNFNK DQQSAFYEIL NMPNLNEEQR NGFIQSLKDD PSQSTNVLGE AKKLNESQA PKC |
| pGE0129 | A-domain | A-domain | T5 | OmpA | AQGT | pJ401 | *VDNNFNKEQQ NAFYEILNMP NLNEEQRNGF IQSLKDDPSQ SANLLAEAKK LNESQAPKC |
| pGE0130 | B-domain | B-domain | T5 | OmpA | AQGT | pJ401 | *VDNKFNKEQQ NAFYEILHLP NLNEEQRNGF IQSLKDDPSQ SANLLAEAKK LNDAQAPKC |
| pGE0131 | C-domain | C-domain | T5 | OmpA | AQGT | pJ401 | *VDNKFNKEQQ NAFYEILHLP NLTEEQRNGF IQSLKDDPSV SKEILAEAKK LNDAQAPKC |

| | | | | | | | |
|---|---|---|---|---|---|---|---|
| *The E, D, A, B and C domains have an A1V mutation to add a restriction site in the start of the sequence (VD) and the D-domain has an inserted D, to have uniform multiple cloning sites of the vectors. | | | | | | | |

**Table 2. Signal peptides.**

| Name | AA sequence | SEQ ID NO |
|---|---|---|
| OmpA | MKKTAIAIAVALAGFATVAQA | 14 |
| DsbA | MKKIWLALAGLVLAFSASA | 15 |

### Cloning of N-terminals

All plasmids were transformed into *E.coli* K12-017 using chemically competent cells. To modify the vectors, a short spacer sequence of 8 AA was inserted between the FspI and KpnI cleave sites. The 8 AA sequences were taken from the first AA in the mature protein of OmpA (AQKDQTWYTGGT, SEQ ID NO 16), DsbA (AQYEDGKQYTGT, SEQ ID NO 17), SpA (AQHDEAQQEAGT, SEQ ID NO 18) or the flexible linker structure from M13 phage GIII AA 236-243 (AQGGGSGGGSGT, SEQ ID NO 19), with modifications in OmpA to fit the restriction site and in OmpA and SpA to mutate asparagines to glutamines (Fig. 1). Moreover, a range of different sequences were cloned into plasmid pGE0002 (Table 1).

### Digestion of plasmids

Plasmids were cleaved with restriction enzymes FspI and KpnI (New England Biolabs (NEB), MA, USA), 6 µl NEB buffer 2.1 was mixed with 6 µg plasmid and 2 µl FspI in total 58 µl. The solution was incubated in 37°C for 1 hour before KpnI was added. The incubation was continued 2 h, followed by addition of 1 µl Calf intestine phosphatase (CIP) followed by 30 min further incubation. Excised bases were removed from the digested plasmids using QIAquick PCR Purification kit (Qiagen, Hilden, Germany).

### Hybridization

Oligonucleotides were ordered from Integrated DNA technologies (IDT, IA, USA). All oligonucleotides were modified with a five prime phosphate group by manufacturer. Two complementary oligonucleotide pairs were mixed in ligation buffer and heated to 95°C, 4 min, followed by cooling down to room temperature. The hybridized fragment was ligated into the FspI and KpnI cleaved plasmids by use of T4 DNA ligase (NEB, MA, USA). A complete list of oligonucleotides used is found in Table 3.

**Table 3. List of oligonucleotides used for addition of N-terminal start sequence.**

| Oligo | Name | Sequence | SEQ ID NO |
|---|---|---|---|
| GEOLI_191 | Nterm_Omp_F | GCAGAAAGATCAGACCTGGTACACCGGCGGTAC | 20 |
| GEOLI_192 | Nterm_Omp_R | CGCCGGTGTACCAGGTCTGATCTTTCTGC | 21 |
| GEOLI_193 | Nterm_SPA_F | GCAGCATGATGAAGCGCAGCAGGAAGCGGGTAC | 22 |
| GEOLI_194 | Nterm_SPA_R | CCGCTTCCTGCTGCGCTTCATCATGCTGC | 23 |
| GEOLI_195 | Nterm_dsbA_F | GCAGTATGAAGATGGCAAACAGTACACCGGTAC | 24 |
| GEOLI_196 | Nterm_dsbA_R | CGGTGTACTGTTTGCCATCTTCATACTGC | 25 |
| GEOLI_197 | Nterm_flex_F | GCAGGGTGGCGGTTCTGGCGGTGGCAGCGGTAC | 26 |
| GEOLI_198 | Nterm_flex_R | CGCTGCCACCGCCAGAACCGCCACCCTGC | 27 |
| GEOLI_284 | Nterm_dsbA7_F | GCAGTATGAAGATGGCAAACAGTACGGTAC | 94 |
| GEOLI_285 | Nterm_dsbA7_R | CGTACTGTTTGCCATCTTCATACTGC | 95 |
| GEOLI_286 | Nterm_dsbA6_F | GCAGTATGAAGATGGCAAACAGGGTAC | 96 |
| GEOLI_287 | Nterm_dsbA6_R | CCTGTTTGCCATCTTCATACTGC | 97 |
| GEOLI_288 | Nterm_dsbA5_F | GCAGTATGAAGATGGCAAAGGTAC | 98 |
| GEOLI_289 | Nterm_dsbA5_R | CTTTGCCATCTTCATACTGC | 99 |
| GEOLI_290 | Nterm_dsbA4_F | GCAGTATGAAGATGGCGGTAC | 100 |
| GEOLI_291 | Nterm_dsbA4_R | CGCCATCTTCATACTGC | 101 |
| GEOLI_292 | Nterm_dsbA8_noGT_F | GCAGTATGAAGATGGCAAACAGTACACCGT | 102 |
| GEOLI_293 | Nterm_dsbA8_noGT_R | CTACGGTGTACTGTTTGCCATCTTCATACTGC | 103 |
| GEOLI_294 | Nterm_dsbA6_noGT_F | GCAGTATGAAGATGGCAAACAGGT | 104 |
| GEOLI_295 | Nterm_dsbA6_noGT_R | CTACCTGTTTGCCATCTTCATACTGC | 105 |
| GEOLI_296 | Nterm_dsbA8_ET_F | GCAGTATGAAGATGGCAAACAGTACACCGAAACC GT | 106 |
| GEOLI_297 | Nterm_dsbA8_ET_R | CTACGGTTTCGGTGTACTGTTTGCCATCTTCATAC TGC | 107 |
| GEOLI_298 | Nterm_dsbA8_DT_F | GCAGTATGAAGATGGCAAACAGTACACCGATACC GT | 108 |
| GEOLI_299 | Nterm_dsbA8_DT_R | CTACGGTATCGGTGTACTGTTTGCCATCTTCATAC TGC | 109 |
| GEOLI_300 | Nterm_dsbA8_AT_F | | 110 |
| GEOLI_301 | Nterm_dsbA8_AT_R | | 111 |
| GEOLI_302 | Nterm_dsbA7_EDT_F | GCAGTATGAAGATGGCAAACAGTACGAAGATACC GT | 112 |
| GEOLI_303 | Nterm_dsbA7_EDT_R | CTACGGTATCTTCGTACTGTTTGCCATCTTCATAC TGC | 113 |
| GEOLI_304 | Nterm_dsbA12_F | GCAGTATGAAGATGGCAAACAGTACACCACCCTG GAAAAGGTAC | 114 |
| GEOLI_305 | Nterm_dsbA12_R | CTTTTTCCAGGGTGGTGTACTGTTTGCCATCTTCA TACTGC | 115 |
| GEOLI_306 | Nterm_dsbA16_F | GCAGTATGAAGATGGCAAACAGTACACCACCCTG GAAAAACCGGTGGCGGGCGGTAC | 116 |
| GEOLI_307 | Nterm_dsbA16_R | CGCCCGCCACCGGTTTTTCCAGGGTGGTGTACTGT TTGCCATCTTCATACTGC | 117 |
| GEOLI_308 | Nterm_R8_F | GCAGCGCCGTCGTCGCCGTCGCCGTCGTGGTAC | 118 |
| GEOLI_309 | Nterm_R8_R | CACGACGGCGACGGCGACGACGGCGCTGC | 119 |
| GEOLI_310 | Nterm_K8_F | GCAGAAGAAGAAAAAGAAGAAGAAAAAGGGTAC | 120 |
| GEOLI_311 | Nterm_K8_R | CCTTTTTCTTCTTCTTTTTCTTCTTCTGC | 121 |
| GEOLI_312 | Nterm_H8_F | GCAGCATCACCACCATCACCATCACCATGGTAC | 122 |
| GEOLI_313 | Nterm_H8_R | CATGGTGATGGTGATGGTGGTGATGCTGC | 123 |
| GEOLI_314 | Nterm_H6_F | GCAGCATCACCACCATCACCATGGTAC | 124 |
| GEOLI_315 | Nterm_H6_R | CATGGTGATGGTGGTGATGCTGC | 125 |
| GEOLI_316 | Nterm_H4_F | GCAGCATCACCACCATGGTAC | 126 |
| GEOLI_317 | Nterm_H4_R | CATGGTGGTGATGCTGC | 127 |
| GEOLI_318 | Nterm_Strep_F | GCAGTGGAGCCATCCGCAGTTTGAAAAAGGTAC | 128 |
| GEOLI_319 | Nterm_Strep_R | /5Phos/CTTTTTCAAACTGCGGATGGCTCCACTGC | 129 |

### Cloned constructs

Ligated plasmids were transformed into chemically competent *E. coli* K12-017 cells. The cells were thawed on ice for 30 min, 100 µl competent cells were added to 10 µl ligation reaction. Cells were incubated on ice for 20 min followed by heat shock at 42°C for 1 min, followed by incubation on ice 5 min. Followed by addition of 900 µl SOC-medium (NEB, MA, USA). Transformation reaction was incubated in 37°C in a rotary shake incubator for 60 min, 100 µl of each reaction was spread on Luria agar plates containing appropriate selective antibiotics. Positive clones were screened with Polymerase chain reaction (PCR), and clones with correct insert were selected. Selected clones were grown in 10 ml Luria broth (LB) over-night (o/n) containing appropriate antibiotics, followed by plasmid preparations using Qiagen Plasmid Miniprep kit (Qiagen, Hilden, Germany). Plasmids were sent to GATC Biotech for sequence verification (GATC Biotech, Cologne, Germany).

**Table 4. Resulting plasmids after addition of N-terminal start sequence.**

| Plasmid number | Insert | Alias | Promoter | Signal peptide | N-terminal start | Cloning vector | Insert sequence |
|---|---|---|---|---|---|---|---|
| pGE0138 | Zvar2₁ | OmpA8AA Zvar2₁ | RhaBAD | DsbA | AQKDQTWYTGGT | pD861-SR | VDAKFDKEAQ EAFYEILHLP NLTEEQRNAF IQSLKDDPSV SKAILAEAKK LNDAQAPKC |
| pGE0139 | Zvar2₁ | SpA8AA-Zvar2₁ | RhaBAD | DsbA | AQHDEAQQEAGT | pD861-SR | VDAKFDKEAQ EAFYEILHLP NLTEEQRNAF IQSLKDDPSV SKAILAEAKK LNDAQAPKC |
| pGE0140 | Zvar2₁ | DsbA8AA-Zvar2₁ | RhaBAD | DsbA | AQYEDGKQYTGT | pD861-SR | VDAKFDKEAQ EAFYEILHLP NLTEEQRNAF IQSLKDDPSV SKAILAEAKK LNDAQAPKC |
| pGE0141 | Zvar2₁ | Flex8AA-Zvar2₁ | RhaBAD | DsbA | AQGGGSGGGSGT | pD861-SR | VDAKFDKEAQ EAFYEILHLP NLTEEQRNAF IQSLKDDPSV |
| | | | | | | | SKAILAEAKK LNDAQAPKC |
| pGE0142 | Zvar2₁ | OmpA8AA-Zvar2₁ | RhaBAD | OmpA | AQKDQTWYTGGT | pD861-SR | VDAKFDKEAQ EAFYEILHLP NLTEEQRNAF IQSLKDDPSV SKAILAEAKK LNDAQAPKC |
| pGE0143 | Zvar2₁ | SpA8AA-Zvar2₁ | RhaBAD | OmpA | AQHDEAQQEAGT | pD861-SR | VDAKFDKEAQ EAFYEILHLP NLTEEQRNAF IQSLKDDPSV SKAILAEAKK LNDAQAPKC |
| pGE0144 | Zvar2₁ | DsbA8AA-Zvar2₁ | RhaBAD | OmpA | AQYEDGKQYTGT | pD861-SR | VDAKFDKEAQ EAFYEILHLP NLTEEQRNAF IQSLKDDPSV SKAILAEAKK LNDAQAPKC |
| pGE0145 | Zvar2₁ | Flex8AA-Zvar2₁ | RhaBAD | OmpA | AQGGGSGGGSGT | pD861-SR | VDAKFDKEAQ EAFYEILHLP NLTEEQRNAF IQSLKDDPSV SKAILAEAKK LNDAQAPKC |
| pGE0180 | Zvar2₆ | DsbA8AA-Zvar2₆ | T5 | OmpA | AQYEDGKQYTGT | pJ401 | (VDAKFDKEAQ EAFYEILHLP NLTEEQRNAF IQSLKDDPSV SKAILAEAKK LNDAQAPK)x6C |
| pGE0293 | E-domain | DsbA8AA-E-domain | T5 | OmpA | AQYEDGKQYTGT | pJ401 | VDEAQQNAFY QVLNMPNLNA DQRNGFIQSL KDDPSQSANV LGEAQKLNDSQ APKC |
| pGE0294 | D-domain | DsbA8AA-D-domain | T5 | OmpA | AQYEDGKQYTGT | pJ401 | VDAQQNNFNK DQQSAFYEIL NMPNLNEEQR NGFIQSLKDD PSQSTNVLGE AKKLNESQA PKC |
| pGE0295 | A-domain | DsbA8AA-A-domain | T5 | OmpA | AQYEDGKQYTGT | pJ401 | VDNNFNKEQQ NAFYEILNMP NLNEEQRNGF IQSLKDDPSQ SANLLAEAKK LNESQAPKC |
| pGE0296 | B-domain | DsbA8AA-B-domain | T5 | OmpA | AQYEDGKQYTGT | pJ401 | VDNKFNKEQQ NAFYEILHLP NLNEEQRNGF IQSLKDDPSQ SANLLAEAKK LNDAQAPKC |
| pGE0297 | C-domain | DsbA8AA-C-domain | T5 | OmpA | AQYEDGKQYTGT | pJ401 | VDNKFNKEQQ NAFYEILHLP NLTEEQRNGF IQSLKDDPSV SKEILAEAKK LNDAQAPKC |
| pGE0298 | Zvar₁_OptExp | DsbA8AA-Zvar₁ | T5 | OmpA | AQYEDGKQYTGT | pJ401 | VDAKFDKEQQ NAFYEILHLP NLTEEQRNAF IQSLKDDPSQ SANLLAEAKK LNDAQAPKC |
| pGE0299 | Zvar2₁ | DsbA8AA-Zvar2₁ | T5 | OmpA | AQYEDGKQYTGT | pJ401 | VDAKFDKEAQ EAFYEILHLP NLTEEQRNAF IQSLKDDPSV SKAILAEAKK LNDAQAPKC |
| pGE0300 | Zvar₄_OptEc | DsbA8AA-Zvar₄ | T5 | OmpA | AQYEDGKQYTGT | pJ401 | (VDAKFDKEQQ NAFYEILHLP NLTEEQRNAF IQSLKDDPSQ SANLLAEAKK LNDAQAPK)x4C |

### Example 1

### Protein expression and purification in shake flasks

*Escherichia coli* strain K12-017 transformed with the recombinant plasmids pGE0138-145 were cultured in 30°C for 4 h in 100 ml Terrific Broth (TB) medium (containing 12g tryptone, 24 g yeast extract, 5 g glycerol (85%), 2,31 g KH₂PO₄, 12,54 g K₂HPO₄ and 50 mg of kanamycin sulphate per liter). Cultures were induced when OD_{600 nm} reached 1-2, using 4 mM L-rhamnose (Sigma Aldrich, MO, USA). Cultures were further incubated in 30°C 17-20 h. Culture solution was subjected to low-speed centrifugation (at 4,000 rpm) for 20 min in a swing out rotor to collect wet cell pellet. The bacterial cell pellets were suspended in 20 ml of a 25 mM phosphate buffer solution (pH 7.4), and the cells were lysed by heat treatment at 85°C for 10 min in a heat block. Followed by a high-speed centrifugation (at 13 000 rpm) for 10 min to separate the supernatant. Then the supernatants were filtered through 0.2 µm syringe filter to remove any residual particles before it was applied to an IgG Sepharose 6FF (GE Healthcare Bio-Sciences, Uppsala, Sweden) affinity chromatography column. The column was equilibrated with loading buffer (25 mM phosphate pH7, 250 mM NaCl) followed by loading of sample. After loading, the column was washed with 5 column volumes (CV) of loading buffer and one CV of low salt wash buffer (50 mM acetate pH6), followed by elution with 50 mM acetic acid, pH2.8. Absorbance at 237 nm was measured in-line using an ÄKTA explorer 100 chromatography system (GE Healthcare Bio-Sciences, Uppsala, Sweden) and peak integration of the eluted peak was performed in the system software (Unicorn 5.1).

### Shake flask results of 2 signal peptides and 4 N-terminal starts

In the first work of optimization of the expression system, two different signal peptides were tested, utilizing different secretion pathways in the cell. DsbA utilizes the signal recognition particle (SRP) pathway and OmpA the Sec pathway. Results from protein expression using DsbA and OmpA signal peptides, showed that the OmpA signal peptide had higher protein expression; 2-5 times higher compared to DsbA signal peptide. Moreover, the first N-terminal AA after the cleave site of the signal peptide had a significant impact on the expression levels. In this study, the DsbA start AA had the highest expression level together with both signal peptides. The lowest expression level was seen for the Flex8AA start sequence using both signal peptides.

### Liquid chromatography coupled mass spectrometry (LC/MS) results

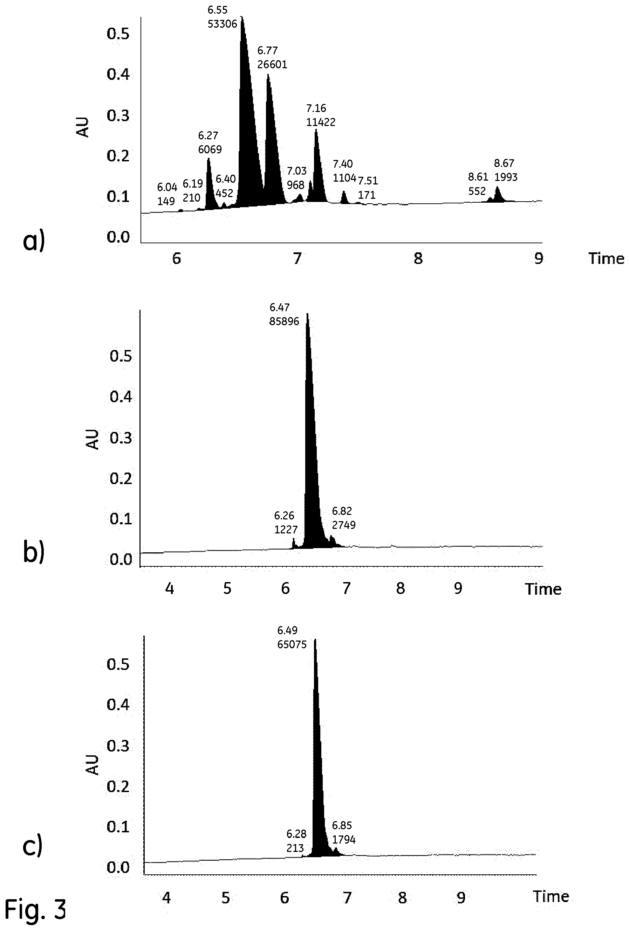

Eluates from IgG Sepharose 6FF were analysed using LC/MS (Waters, PA, USA). Results showed that the eluate from the reference construct pGE0120, with AQGT as N-terminal start sequence had a range of different signal peptide cleave sites (Fig. 3; a). Integrated area of peak with correct mass was 52% of the total area with correctly processed signal peptide. Other peaks corresponded to cleave sites with 6 AA extra, 9 AA extra, intact signal peptide (21 AA extra) and 7 AA missing from the main construct. After addition of 8-AA from DsbA, the signal peptide was cleaved correctly to 96% (Fig. 3; b). A similar result was seen with DsbA signal peptide and DsbA 8AA N-terminal, with 97% correctly cleaved signal peptide.

### Example 2

### Protein expression in fed-batch fermenter

Six, 1 L working volume fermenters, GRETA (Belach Bioteknik, Skogås, Sweden) were used. Starting volume was set to 750 ml, and end volume was approximately 1 L. Aeration was set to 1 L/min, temperature, pH and antifoam were controlled automatically. pH was kept at set value by the addition of 25% ammonia and 2 M phosphoric acid. Antifoam control was automatically adding Breox FMT 30 (BASF, Ludwigshafen, Germany), when high foaming occurred. pH and dissolved oxygen (DO) were controlled with probes from Broadley James (CA, USA). DO was kept constant at 30% by increasing stirrer speed from 300 rpm up to 1500 rpm. When the stirrer reached max speed, DO second set point was 20%, and was held constant by addition of mixing pure oxygen into the airflow. Terrific Broth (TB) supplemented with 50 mg/L kanamycin or neomycin was used in the shake flask pre-cultivations and was started by addition of 100 µl cell suspension to 100 mL TB supplemented with 50 µg/mL kanamycin or neomycin and incubated in 37°C for 17 h. Main fermentation media was inoculated with 10 ml pre-cultivation. Glucose feed with 60% (w/v) (VWR, PA, USA) was fed into the fermenter according to a pre-set profile after initial batch glucose was consumed. Total length of the main fermentation was 26 h.

### Concentration analysis using standard curve

Fermentation samples were heat treated at 85°C for 5 min in 1.5 ml tubes. Followed by high speed centrifugation (at 13500 rpm, in benchtop centrifuge) for 5 min to separate the supernatant. Supernatants were filtered through 0.2 µm syringe filter to remove any residual particles before they were applied to an IgG Sepharose 6FF HiTrap^{™} column (GE Healthcare Bio-Sciences, Uppsala Sweden). The column was equilibrated with loading buffer (Phosphate buffered Saline (Medicago, Uppsala, Sweden)) followed by loading of 50 µl sample. After loading, the column was washed with 5 CV of loading buffer, followed by elution with 200 mM phosphate buffer, pH2.9. Absorbance at 237 nm was measured in-line and peak integration of the eluted peak was performed in the chromatography system software. Protein concentration of the samples were determined by a standard curve using a purified protein of known concentration.

### Fermentation, protein expression results

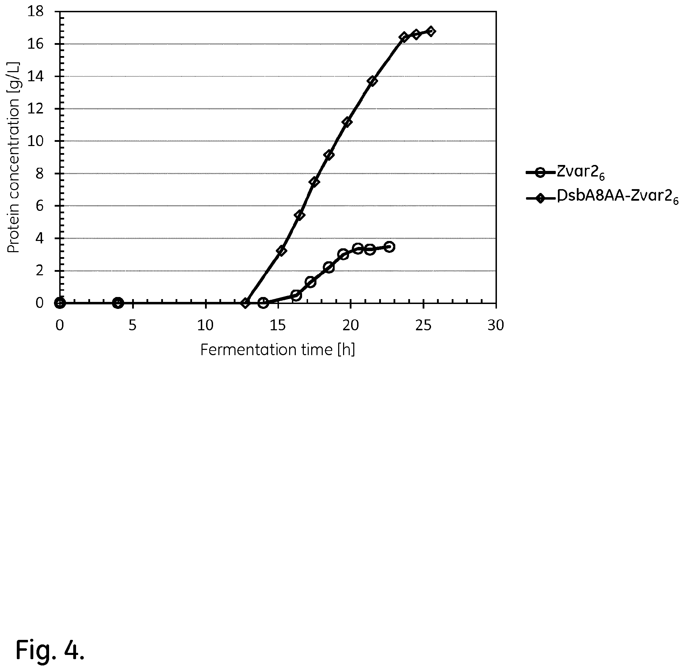

Plasmid pGE0002 containing Zvar2 hexamer (Zvar2₆) was cleaved with FspI and KpnI restriction enzymes. The N-terminal start from DsbA was ligated into the cleaved vector, and the resulting plasmid was denoted pGE0180. These two vectors were transformed into *E.coli* K12-017 and the resulting constructs were expressed in fed-batch fermentations to see differences in protein expression and signal peptide cleavage patterns. Protein concentration analysis using standard curve showed that the protein expression increased from 3.5 g/L to 16.8 g/L, i.e. a more than 4 times increase in protein expression (Fig. 4).

### LC/MS results

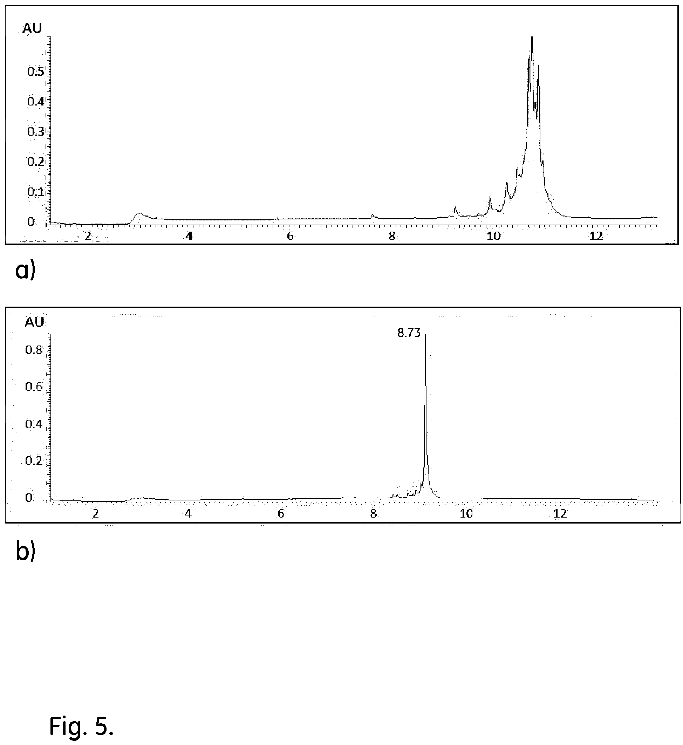

Eluates from IgG Sepharose were analysed using LC/MS (Waters, PA, USA). The results showed that the eluate from the reference construct pGE0002 showed a range of different signal peptide sites including cleave sites with extra 6 AA and 9 AA, previously seen in experiment 1 (Fig. 5, a). However, when adding 8-AA from DsbA on the N-terminus the signal peptide was cleaved correctly, resulting in a clear singular peak of correct mass (Fig. 5; b).

### Example 3

Protein expression in fed-batch fermenter and concentration analysis using standard curve performed as in Example 2.

### Protein preparation

Filtered samples (See above) were applied to an IgG Sepharose 6FF Tricorn 10 column with a CV of 10.5 ml (GE Healthcare Bio-Sciences, Uppsala, Sweden). The column was equilibrated with high salt loading buffer (50 mM phosphate, pH7.0, 500 mm NaCl) followed by loading of 0.5-5 mL sample based on concentration calculations (See above). After loading, the column was washed with 5 CV of loading buffer, followed by elution with 100 mM acetic acid. Eluted samples were collected and concentrated to approximately 1 mg/ml using Vivaspin 5, 3000 Da cut-off (GE Healthcare Bio-Sciences, Uppsala, Sweden). The amount of protein was estimated by absorbance measurements at 280 nm using specific absorbance coefficients, calculated based on Pace et al. Protein Science 4, 2411-2423, (1995) and Beer Lamberts law. Sample concentration was back-calculated using total protein mass in elution pool and volume of injected sample. The concentration was confirmed by amino acid analysis. The homogeneity and the molecular weights were analysed using mass spectrometry on a Waters Q-Tof (PA, USA).

### Protein expression results of additional constructs

To determine the effects of N-terminal start sequence on other domains from *Staphylococcus aureus* protein A and alkali stabilized domain Zvar monomer (Zvar₁) and tetramer (Zvar₄), new plasmids were constructed with the additional N-terminal and expressed in fed-batch fermentations using plasmid pJ401. The results showed that E-domain had no measurable expression without insert. However, with 8AA DsbA start, protein expression was measured to 2.9 g/L. Both the D -and A-domain showed a low expression that was not improved by adding N-terminal start. Both C -and B-domain (which have the same AA sequence in the start) showed very similar expression, with a clear increase in expression with 8AA DsbA start. However, the largest increase of expression was seen on Zvar -and Zvar2 monomer (Zvar₁ and Zvar2₁) with more than 4 times and 10 times protein expression respectively. Also, Zvar tetramer (Zvar₄) showed a significant increase in protein expression with 8AA DsbA start, even though the increase was not as dramatic as for the monomer.

**Table 5. Protein expression of example proteins, all domains of prA, Zvar and Zvar tetramer, with and without N-terminal start sequence.**

| Plasmid | Domain | N-terminal | Concentration [g/L] | Analysis Method* |
|---|---|---|---|---|
| pGE0127 | E-domain | AQGT | 0.0 | Protein preparation |
| pGE0293 | E-domain | 8AA DsbA | 2.9 | Standard curve |
| pGE0128 | D-domain | AQGT | 0.6 | Protein preparation |
| pGE0294 | D-domain | 8AA DsbA | 0.1 | Protein preparation |
| pGE0129 | A-Domain | AQGT | 1.9 | Protein preparation |
| pGE0295 | A-Domain | 8AA DsbA | 0.2 | Protein preparation |
| pGE0130 | B-domain | AQGT | 2.5 | Standard curve |
| pGE0296 | B-domain | 8AA DsbA | 6.3 | Standard curve |
| pGE0131 | C-domain | AQGT | 2.5 | Standard curve |
| pGE0297 | C-domain | 8AA DsbA | 5.8 | Standard curve |
| pGE0132 | Zvar₁ | AQGT | 2.5 | Standard curve |
| pGE0298 | Zvar₁ | 8AA DsbA | 10.2 | Standard curve |
| pGE0120 | Zvar2₁ | AQGT | 0.7 | Protein preparation |
| pGE0299 | Zvar2₁ | 8AA DsbA | 7.3 | Standard curve |
| pGE0091 | Zvar_{4_}OptEc | AQGT | 9.6 | Standard curve |
| pGE0300 | Zvar_{4_}OptEc | 8AA DsbA | 15.3 | Standard curve |

| | | | | |
|---|---|---|---|---|
| *Either the standard curve or protein preparation method, as described above. | | | | |

In the first experiment with N-terminal spacer, two different signal peptides from two secretion pathways were tested, and it was shown that OmpA signal peptide, utilizing Sec dependent pathway had the highest expression and signal peptide cleavage in this experiment. Other signal peptides using this pathway are for example PhoA *(E. coli* alkaline phosphatase), MalE *(E. coli* maltose binding protein) and PelB *(Erwinia carotovora* Pectate lyase B ). On the contrary, the reference construct (with AQGT start) had higher protein expression and signal peptide cleavage using DsbA signal peptide, indicating that it is a combination of signal peptide and N-terminus. Furthermore, four different N-terminals were tested and all worked better compared to the reference construct using OmpA signal peptide. Surprisingly, when the signal peptide cleavage worked optimally, also protein expression was increased, indicating that signal peptidase cleavage might be the rate limiting enzyme in the transport of proteins over the inner membrane. Without being bound by theory, a hypothesis is that protein "gets stuck" in the pores of the inner membrane and halts further expression when secretion is not working optimally. In cultivations of high protein expression, more than 50% of the protein was found outside of the cells in fermentation broth indicating periplasmic leakage to extracellular space. When other domains from *Staphylococcus aureus* protein A were tested, it was seen that the E-domain had no measurable protein expression, which was unexpected given that it is the first domain after the signal peptide in native *Staphylococcus aureus* protein A. However, one important difference is that the first AA after cleave site have been changed from AQHDEA to AQGTVDEA for cloning purposes, and it might be this substitution/insert that affects the signal peptide function negatively. Another difference is that the signal peptide has been substituted from native SpA to OmpA, this in combination with N-terminal start might not be optimal. When adding 8AA DsbA N-terminus to the E-domain in combination with the OmpA signal peptide, protein transport and expression function properly. Moreover, the A and D domains that are very closely related in the N-terminal proximal structural unit express poorly and are not improved by additional AA, whereas the B and C-domains, identical in this unit, express well with the AQGT start, and expression increases additionally when 8AA DsbA are added in the N-terminus. Also, Zvar as monomer and tetramer increase expression with additional AA. However, the most dramatical increase is seen in Zvar2 monomer where protein expression goes from 0.68 g/L to 7.28 g/L. Without being bound by theory, a hypothesis of the difference between Zvar and Zvar2 is that Zvar2 has two mutations in the first alpha helix in position Q9 and N11, which is estimated to increase alpha-helix structure in the start of the protein, as calculated with algorithms such as GORIV, available on-line at https://npsa-prabi.ibcp.fr/cgi-bin/npsa_automat.pl?page=/NPSA/npsa_gor4.html
(PRABI, Rhône-Alpes Bioinformatics Center), thus increasing steric hindrance of the signal peptidase to cleave the signal peptide of the nascent protein.

### Example 4

### Variants of the N-terminal spacer

The N-terminal inserts were each designed to investigate the effect of the insert sequence on one or more of the following parameters: protein yield, signal peptide cleavage and alkaline stability. The designs can be divided into four separate categories as follows:
1. Varying length of existing insert without modifications.
2. Removing suspected site of truncation (glycine-threonine) at alkaline conditions, while varying length of remaining insert.
3. Substituting the glycine in the known site of truncation for various other residues.
4. Substituting the entire sequence.

The aim of category 1 was mainly to investigate the effect of the insert length on signal peptide cleavage and protein yield, and did not explicitly address the suspected truncation site at alkaline conditions. Categories 2 and 3 were mostly aimed at addressing the alkaline stability issue, although they also provide some more data for the other two issues. Category 4 was a more open category, providing a wider set of sequences to provide data in all three areas of interest.

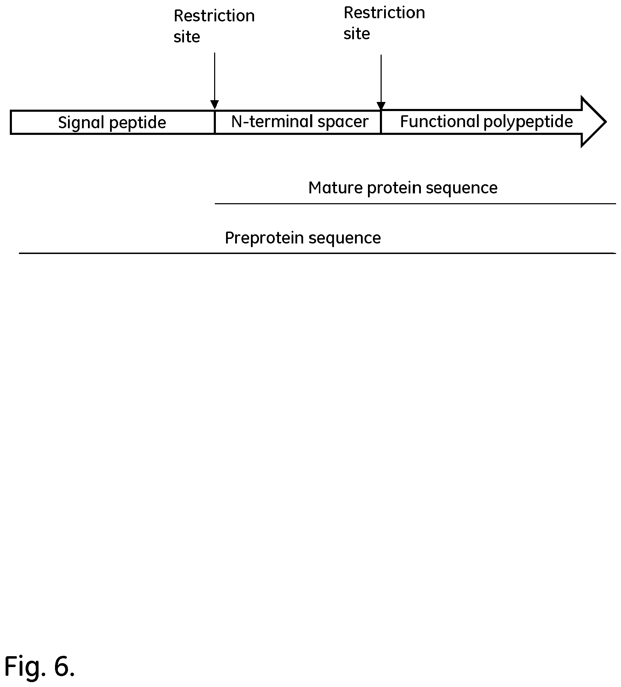

The nucleotide sequences were codon optimized for *E. coli,* but with some degeneracy added in the more repetitive sequences. Inserts belonging to category 1 and 4 were designed to be complementary to the digestion sites of FspI in the 5' end, and KpnI in the 3' end. Since the glycine-threonine truncation site was a part of the KpnI restriction site, inserts belonging to categories 2 and 3 were designed to instead be complementary to the adjacent AccI restriction site in the 3' end, while retaining the 5' complementarity to FspI. The amino acid sequences of all inserts are shown in table 6. A schematic illustration of the general construct is shown in Fig 6.

**Table 6. All inserts used in the experiments. Residues resulting from the restriction site "scar" are marked in italics.**

| **Category** | **Construct name** | **Insert amino acid sequence** | **SEQ ID NO** |
|---|---|---|---|
| Original construct without DsbA insert | pGE0002 | *AQGT* | 28 |
| Original construct with 8 aa DsbA insert | pGE0180 | *AQ*YEDGKQYT*GT* | 17 |
| 1 | DsbA7 | *AQ*YEDGKQY*GT* | 29 |
| | DsbA6 | *AQ*YEDGKQ*GT* | 30 |
| | DsbA5 | *AQ*YEDGK*GT* | 31 |
| | DsbA4 | *AQ*YEDG*GT* | 32 |
| | DsbA12 | *AQ*YEDGKQYTTLEK*GT* | 33 |
| | DsbA16 | *AQ*YEDGKQYTTLEKPVAG*GT* | 34 |
| 2 | DsbA8_noG T | *AQ*YEDGKQYT | 35 |
| | DsbA6_noG T | *AQ*YEDGKQ | 36 |
| 3 | DsbA8_ET | *AQ*YEDGKQYTET | 37 |
| | DsbA8_DT | *AQ*YEDGKQYTDT | 38 |
| | DsbA8_AT | *AQ*YEDGKQYTAT | 39 |
| | DsbA7_EDT | *AQ*YEDGKQYEDT | 40 |
| 4 | R8 | *AQ*RRRRRRRR*GT* | 41 |
| | K8 | *AQ*KKKKKKKK*GT* | 42 |
| | H8 | *AQ*HHHHHHHH*GT* | 43 |
| | H6 | *AQ*HHHHHH*GT* | 44 |
| | H4 | AQ*HHHHGT* | 45 |
| | Strep | *AQ*WSHPQFEK*GT* | 46 |
| | SPA | *AQ*HDEAQQEA*GT* | 47 |

The constructs were assembled by ligating different hybridized oligonucleotides into the plasmid of either pGE0002 or pGE0180, digested with the relevant restriction enzymes to remove the original N-terminal insert. After ligation, the plasmids were transformed into *E. coli* K12-017 cells. Positive colonies were screened using colony PCR and electrophoresis.

Two or three clones from each construct were chosen and sent to GATC biotech (Cologne, Germany) for sequence verification.

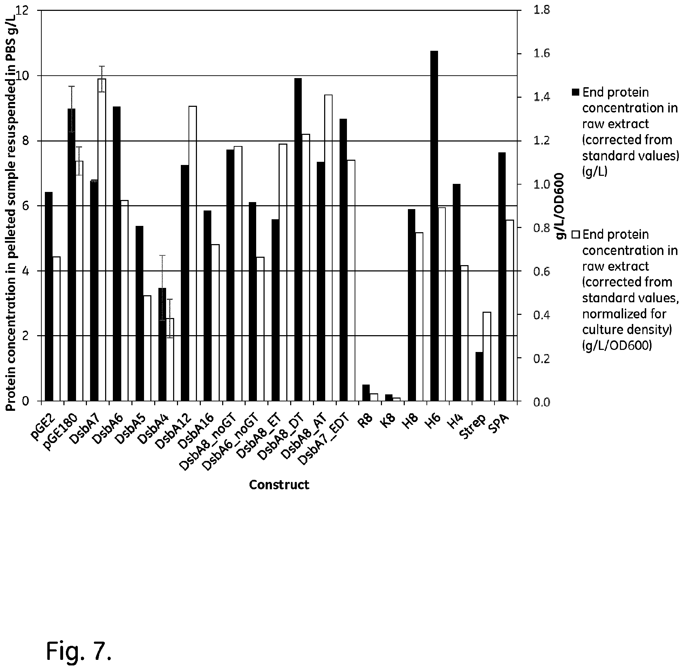

One sequence-verified clone for each construct was selected for protein expression in shake flask cultivation. Figure 7 shows a summary of the results from the quantification of protein yields in the different shake flask cultures done with IgG Sepharose 6FF. For each construct, the left bar is the protein concentration after linear correction using a 5 g/L and a 10 g/L standard solution as reference. The right bar is this concentration divided by the OD₆₀₀ nm at the end of the cultivation. This value is meant to give an idea of whether a low protein concentration is due to an actual low rate of protein production per cell, or simply due to a low culture density. Note that the concentrations are measured after pelleting the cells and re-suspending them in Phosphate buffered saline (Medicago, Uppsala, Sweden), and as such are not directly comparable to concentrations from samples taken from e.g. the multifermenter.

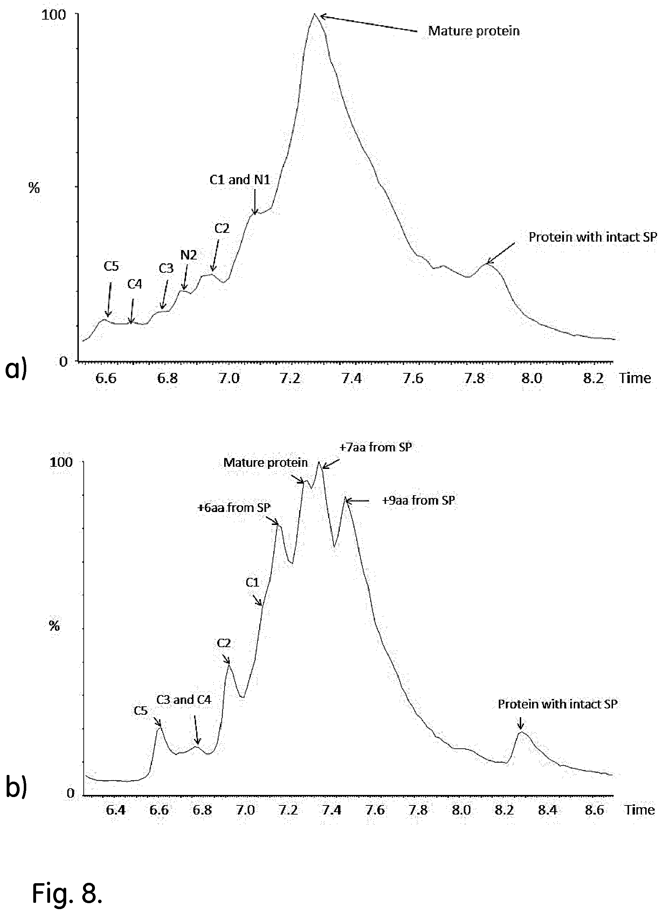

The protein solutions for each construct yielded from the shake flask cultivation were purified using IgG Sepharose 6FF (GE Healthcare, Uppsala, Sweden). The purified protein samples were investigated for signal peptide cleavage using LC/MS analysis (Waters, PA, USA). A protein with a correctly cleaved signal peptide only has one main mass (example Total Ion Count (TIC) shown in Figure 8 a), while a protein with an incorrectly cleaved signal peptide yields one or more secondary peaks (as shown in Figure 8 b). The side peaks further to the left and right of the main peaks are likely due to leakage from the cytoplasm during the heat treatment-step when extracting the protein from the cell culture. The peaks C1 to C5, N1 and N2 are likely different partially digested versions of the protein from the cytoplasm. The small peak to the right of the main peak is protein with uncleaved signal peptide, which would be reasonable to see if leakage from the cytoplasm had occurred.

In many cases, the peaks were hard to interpret conclusively due to the purity of the samples and insufficient separation during the liquid chromatography step. However, it could be seen that the following constructs had a correct signal peptide cleavage: pGE0180, DsbA7, DsbA6, DsbA8noGT, DsbA8_DT, DsbA8_ET, DsbA8_AT, DsbA7_EDT, DsbA12, DsbA16, H8, H6 and SPA.

After the signal peptide cleavage investigation, the protein solutions of constructs with correct signal peptide cleavage were further purified using a Capto Q ImpRes anion exchange column (GE Healthcare, Uppsala, Sweden). Purified samples were subjected to treatment with 1 M sodium hydroxide (NaOH) for 0, 4 and 24 hours. Vivaspin columns (GE Healthcare, Uppsala, Sweden) were used to separate the main protein from any small peptides that might have been truncated. Both the peptide samples and the main proteins were investigated using LC/MS (Waters, PA, USA).

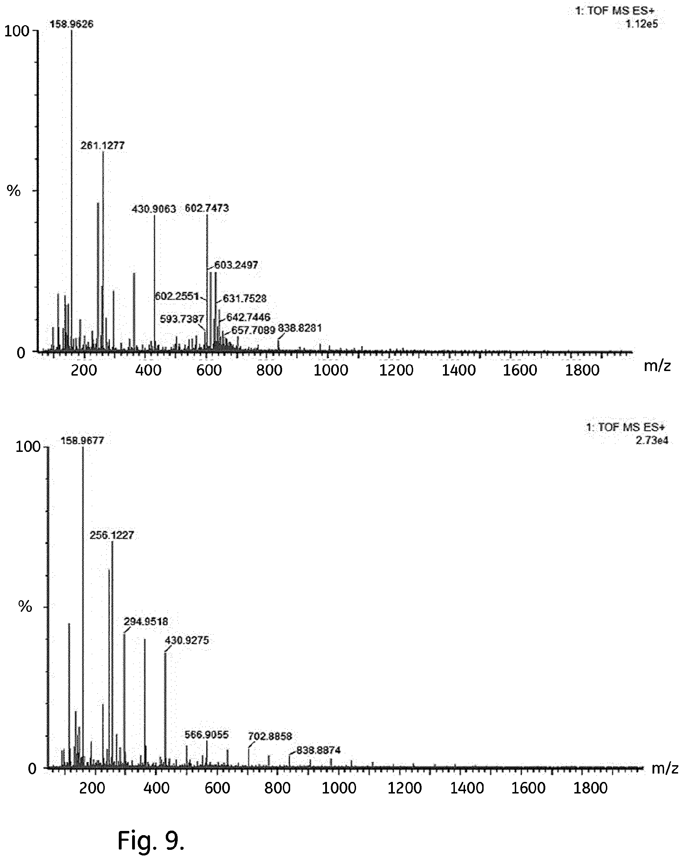

Peptides appeared in the mass spectra with +2 charge, that is, at a mass over charge (m/z) -value corresponding to half their molecular weight. In the cases investigated here, the peptides all had a molecular weight between about 800 Da and 1600 Da. As such, the relevant cluster of peaks appeared between 400 and 800 m/z, as can be seen for pGE0180 to the left in Fig 9.

If peptides were present, the MassLynx search function was used on the TIC to extract the peaks of different peptide masses as extracted ion chromatograms (XIC). The XIC peaks were integrated and the integrated areas were then used to compare the amounts of different peptides in the samples.

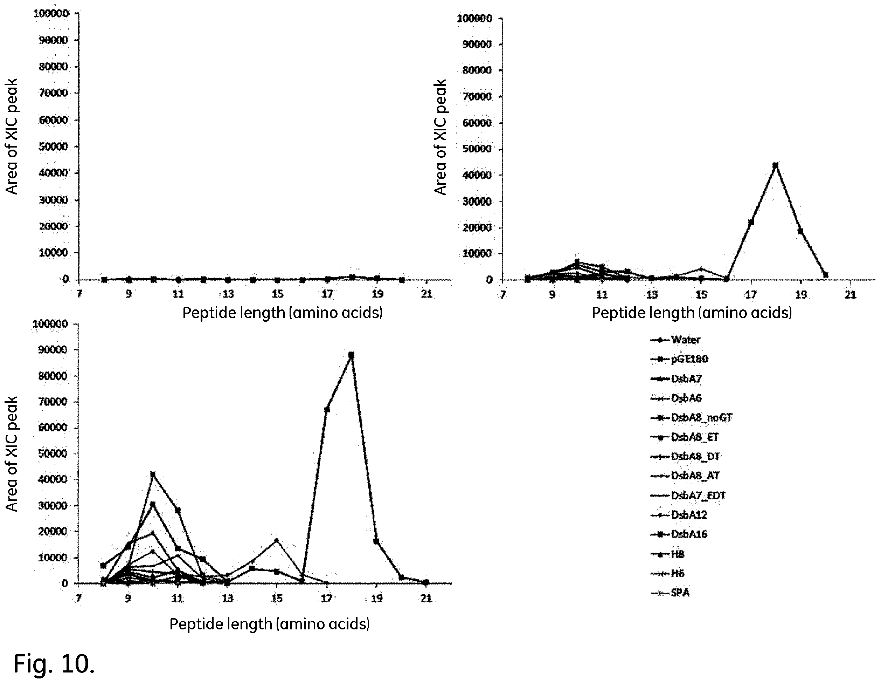

Fig 10 shows a summary of the results from the initial alkaline stability study done with unwashed Vivaspin columns. Despite quite a bit of overlapping background noise, clear patterns are visible after 24 hours of incubation.

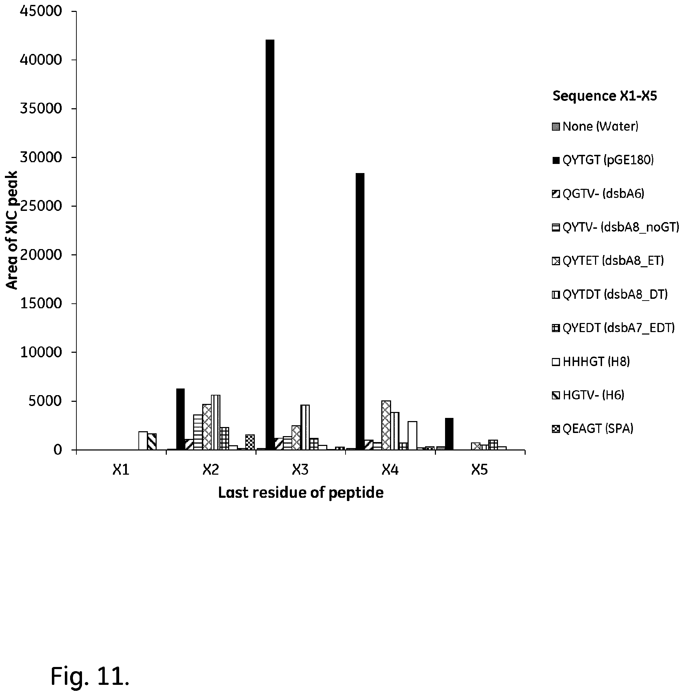

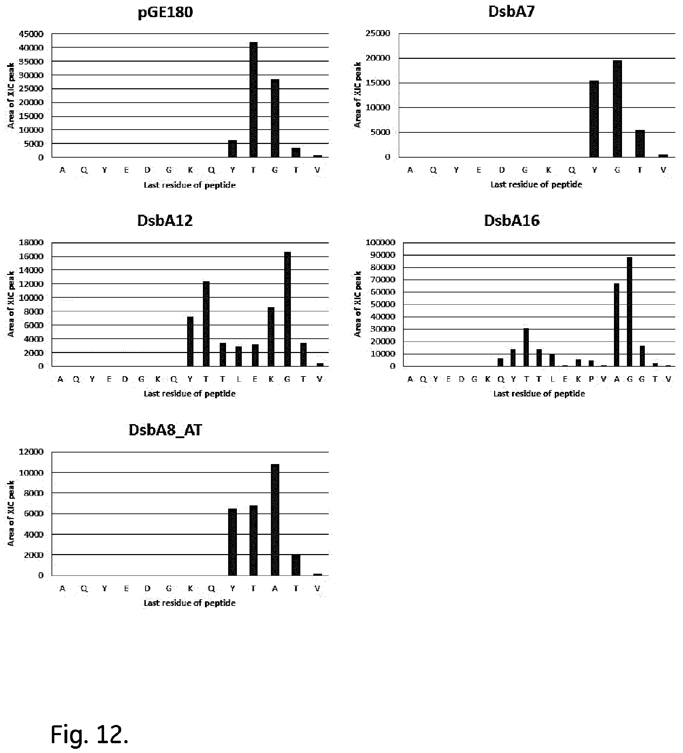

Fig 11 shows a different view of the same data for the most promising constructs (including pGE0180 as a reference), while Fig 12 gives a breakdown of the most prominently cleaved constructs. The sequences around the cleavage site are included to give an idea of what residues might be vulnerable. Notably, for the low peak areas (around 5000 area units), background noise has a significant influence.

The second analysis with spin columns washed with isopropanol yielded mostly similar results to the initial run, although the peak areas were reduced. The notable exception is DsbA6, which was revealed to have some cleavage. This was not immediately detectable from the peptide analysis, but became apparent when investigating the retentate, where DsbA6 showed a clear truncated peak in much the same manner as pGE0180, while DsbA8_noGT and DsbA_DT showed no such peak.

### Example 5

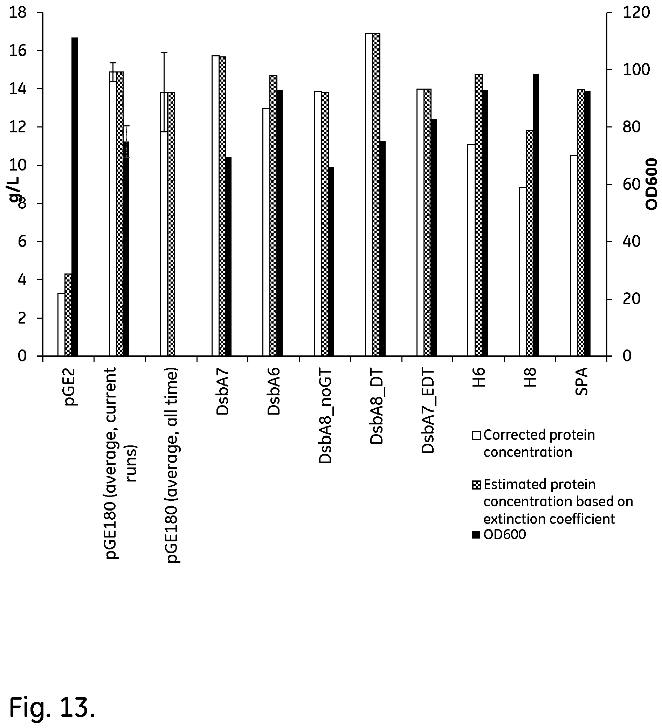

Interesting constructs from the shake-flask study and subsequent analyses of the purified protein were selected for a larger scale cultivation in a multifermenter (Belach Bioteknik, Skogås, Sweden). At the end of the fermentation, the OD₆₀₀ nm and protein concentration were measured (see Fig 13).

Since the different proteins have somewhat varying extinction coefficients, it stands to reason that they might vary in absorbance at 237 nm as well, which is where the protein concentration was estimated. Therefore, the ratio between the pGE0180 extinction coefficient (0.294) and the calculated extinction coefficient for each other protein was used to estimate a more "true" value.

The fed-batch cultures of constructs DsbA8_noGT and DsbA8_DT were heat treated to extract periplasmic protein, and centrifuged and diafiltered to remove cellular debris. The filtrate was conditioned to remove some contaminants via precipitation, and prepare the solution for purification with Capto S ImpAct (GE Healthcare, Uppsala, Sweden) using a pH gradient. The elute was reduced to remove dimers, and desalted to remove the DTT and get the correct buffer for the anion exchange purification, where a salt gradient was used. The final purified solution was concentrated via membrane ultrafiltration to reach the concentration needed for immobilization at the desired ligand densities. The end product was analysed with SEC for purity and LC/MS for molecular weight, with satisfactory results.

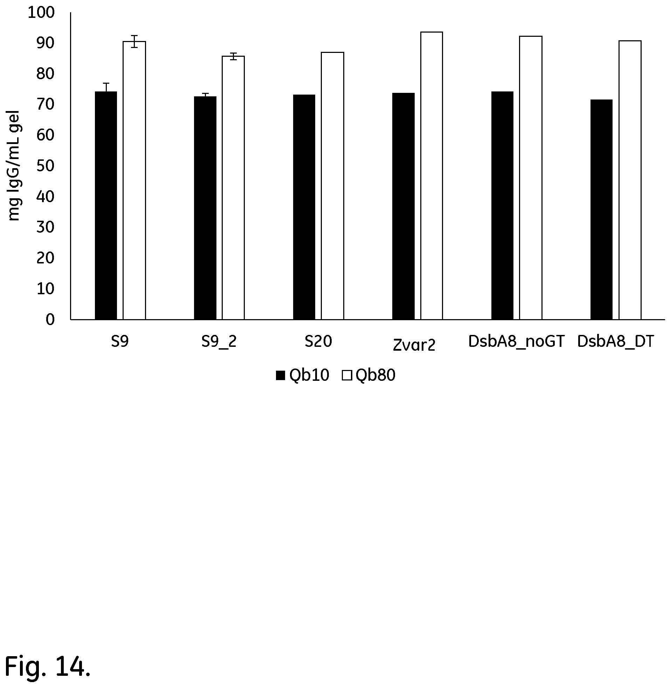

The purified proteins (>90% purity) were coupled to highly cross-linked agarose beads, aiming for ligand densities similar to those in a previous experiment where Zvar2 was immobilized on a base matrix of similar properties and analyzed for dynamic binding capacity. The coupled gels were packed in Tricorn 5/100 columns (GE Healthcare, Uppsala, Sweden). The packing of the gel bed was evaluated through the asymmetry of an acetone peak. The packed columns were used to measure the dynamic binding capacity of the gels using IgG. One measurement per construct was made. The results are summarized in table 7. Fig 14 shows a comparison between measurements done in this instance, and those done in the aforementioned previous experiment.

**Table 7. Asymmetry and dynamic binding capacity values for the different protein-coupled gels.**

| | **Zvar2** | **DsbA8_noGT** | **DsbA8_DT** |
|---|---|---|---|
| Qb10 (mg IgG/mL gel) | 73.7 | 74.2 | 71.6 |
| Qb80 (mg IgG/mL gel) | 93.6 | 92.2 | 90.7 |

### Example 6

The N-terminal spacer AQYEDGKQYTGT and a C-terminal cysteine was introduced in a number of monomeric and dimeric further mutants of Zvar2 (as listed below), using the OmpA signal peptide. The structure of the mature protein was thus: AQYEDGKQYTGT-IgG-binding polypeptide-C.

Plasmids were transformed into chemically *competent E.coli* K12-017 cells. The cells were thawed on ice for 30 min, 50 µl competent cells were added to 20 ng plasmids. Cells were incubated on ice for 20 min followed by heat shock at 42°C for 1 min, followed by incubation on ice 5 min and addition of 400 µl SOC-medium (NEB, MA, USA). Transformation reaction was incubated in 37°C in a rotary shake incubator for 60 min, 200 µl of each reaction was spread on Luria agar plates containing appropriate selective antibiotics.

*E. coli* strain K12-017 transformed with the recombinant plasmids (Table 8) were cultured in 37°C in 100 ml Terrific Broth (TB) medium (containing 12g tryptone, 24 g yeast extract, 5 g glycerol (85%), 2,31 g KH₂PO₄, 12,54 g K₂HPO₄ and 50 mg of kanamycin sulphate per liter). Cultures were induced when OD_{600 nm} reached 1, using 1 mM IPTG (Sigma Aldrich, MO, USA). Cultures were further incubated in 30°C 17-20 h. Culture solution was subjected to low-speed centrifugation (at 4,000 rpm) for 20 min in a swing out rotor to collect wet cell pellet. The bacterial cell pellets were suspended in 10 ml of a 25 mM phosphate buffer solution (pH 7.4), and the cells were lysed by heat treatment at 85°C for 15 min in a heat block. Followed by a high-speed centrifugation (at 10 000 x g) for 10 min to separate the supernatant. Then supernatants were filtered through 0.2 µm syringe filter to remove any residual particles before it was applied to an XK 16-6 IgG Sepharose 6FF (GE Healthcare Bio-Sciences, Uppsala, Sweden). The column was equilibrated with loading buffer (25 mM phosphate pH7, 250 mM NaCl) followed by loading of sample. After loading the column was washed with 5 column volumes (CV) of loading buffer and one CV of low salt wash buffer (50 mM acetate pH6), followed by elution with 50 mM acetic acid, pH2.8. Absorbance at 237 nm was measured in-line using an ÄKTA explorer 100 (GE Healthcare Bio-Sciences, Uppsala, Sweden) and peak integration of the eluted peak was performed in the system software (Unicorn 5.1).

The IgG-binding fraction was eluted and collected in a pool and the volumes and protein concentrations of the pools were noted - see table 8. The amount of IgG-binding protein expressed may in several cases be higher than the amount recovered, due to loss of protein secreted into the culture medium and/or to overloading of the IgG column.

**Table 8. Expression of IgG-binding proteins with N-terminal spacer AQYEDGKQYTGT and with a C-terminal cysteine.**

| IgG-binding protein | SEQ ID NO | Protein conc. mg/mL | Pool volume, mL | Recovered protein mg |
|---|---|---|---|---|
| Zvar2(A29G)₁ | 48 | 1.4 | 3 | 4.2 |
| Zvar2 (A29S)₁ | 49 | 0.8 | 3 | 2.4 |
| Zvar2 (A29Y)₁ | 50 | 2.0 | 4 | 8.0 |
| Zvar2 (A29Q)₁ | 51 | 2.3 | 3 | 6.9 |
| Zvar2 (A29T)₁ | 52 | 2.1 | 3 | 6.3 |
| Zvar2 (A29N)₁ | 53 | 2.0 | 4 | 8.0 |
| Zvar2 (A29F)₁ | 54 | 2.0 | 4 | 8.0 |
| Zvar2 (A29L)₁ | 55 | 2.3 | 3 | 6.9 |
| Zvar2 (A29W)₁ | 56 | 2.1 | 3 | 6.3 |
| Zvar2 (A29I)₁ | 57 | 1.6 | 3 | 4.8 |
| Zvar2 (A29M)₁ | 58 | 2.0 | 4 | 8.0 |
| Zvar2 (A29V)₁ | 59 | 2.1 | 3 | 6.3 |
| Zvar2 (A29D)₁ | 60 | 2.3 | 3 | 6.9 |
| Zvar2 (A29E)₁ | 61 | 2.2 | 4 | 8.8 |
| Zvar2 (A29H)₁ | 62 | 1.2 | 4 | 4.8 |
| Zvar2 (A29R)₁ | 63 | 1.9 | 4 | 7.6 |
| Zvar2 (A29K)₁ | 64 | 2.0 | 4 | 8.0 |
| Zvar2 (Δ235,236,237)₂ | 65 | 2.2 | 4 | 8.8 |
| Zvar2 (Δ233,234,235)₂ | 66 | 2.9 | 4 | 11.6 |
| Zvar2 (Δ5-1)₂ | 67 | 2.1 | 5 | 10.5 |
| Zvar2 (Δ5-2)₂ | 68 | 2.8 | 6 | 16.8 |
| Zvar2 (D3C-term)₂ | 69 | 3.3 | 5 | 16.5 |
| Zvar2 (D3N-term)₂ | 70 | 3.5 | 2.7 | 9.4 |
| Zvar2 (D8N-term)₂ | 71 | 2.6 | 2.1 | 5.46 |
| Zvar2 (Linker+8)₂ | 72 | 3.0 | 3.4 | 10.2 |
| Zvar2 (Linker+4)₂ | 73 | 3.0 | 2.7 | 8.1 |
| Zvar2 (Δ Q9)₁ | 74 | 1.4 | 1.7 | 2.4 |
| Zvar2 (Δ Q40)₁ | 75 | 2.2 | 3.7 | 8.1 |
| Zvar2 (Δ A42)₁ | 76 | 1.7 | 1.7 | 2.9 |
| Zvar2 (Δ N43)₁ | 77 | 1.6 | 2 | 3.2 |
| Zvar2 (Δ L44)₁ | 78 | 1.3 | 1.2 | 1.6 |
| Zvar2 (E11N,A12F)₁ | 79 | 1.3 | 1.1 | 1.4 |
| Zvar2 (E11N,A12Y)₁ | 80 | 1.2 | 0.75 | 0.9 |
| Zvar2 (E11N,A12K)₁ | 81 | 1.4 | 0.72 | 1.0 |
| Zvar2 (E11N,A12R)₁ | 82 | 1.8 | 1.49 | 2.7 |
| Zvar2 (L22F)₁ | 83 | 2.9 | 2.77 | 8.0 |
| Zvar2 (A43N,I44F)₁ | 84 | 1.9 | 4 | 7.6 |
| Zvar2 (A43N,I44Y)₁ | 85 | 2,4 | 3 | 7.2 |
| Zvar2 (A43N,I44W)₁ | 86 | 1.1 | 3 | 3.3 |
| Zvar2 (A43N,I44R)₁ | 87 | 2.4 | 3.9 | 9.4 |
| Zvar2 (A43N,I44K)₁ | 88 | 2.4 | 4 | 9.6 |
| Zvar2 (D53F)₁ | 89 | 2.7 | 3 | 8.1 |
| Zvar2 (D53Y)₁ | 90 | 2.8 | 3 | 8.4 |
| Zvar2 (D53W)₁ | 91 | 1.3 | 2.9 | 3.8 |
| Zvar2 (D53K)₁ | 92 | 2.2 | 4 | 8.8 |
| Zvar2 (D53R)₁ | 93 | 2.2 | 3 | 6.6 |

Zvar2(A29G) monomer (SEQ ID NO 48)
   VDAKFDKEAQEAFYEILHLPNLTEEQRNGFIQSLKDDPSVSKAILAEAKKLNDAQAPK
Zvar2 (A29S) monomer (SEQ ID NO 49)
   VDAKFDKEAQEAFYEILHLPNLTEEQRNSFIQSLKDDPSVSKAILAEAKKLNDAQAPK
Zvar2 (A29Y) monomer (SEQ ID NO 50)
   VDAKFDKEAQEAFYEILHLPNLTEEQRNYFIQSLKDDPSVSKAILAEAKKLNDAQAPK
Zvar2 (A29Q) monomer (SEQ ID NO 51)
   VDAKFDKEAQEAFYEILHLPNLTEEQRNQFIQSLKDDPSVSKAILAEAKKLNDAQAPK
Zvar2 (A29T) monomer (SEQ ID NO 52)
   VDAKFDKEAQEAFYEILHLPNLTEEQRNTFIQSLKDDPSVSKAILAEAKKLNDAQAPK
Zvar2 (A29N) monomer (SEQ ID NO 53)
   VDAKFDKEAQEAFYEILHLPNLTEEQRNNFIQSLKDDPSVSKAILAEAKKLNDAQAPK
Zvar2 (A29F) monomer (SEQ ID NO 54)
   VDAKFDKEAQEAFYEILHLPNLTEEQRNFFIQSLKDDPSVSKAILAEAKKLNDAQAPK
Zvar2 (A29L) monomer (SEQ ID NO 55)
   VDAKFDKEAQEAFYEILHLPNLTEEQRNLFIQSLKDDPSVSKAILAEAKKLNDAQAPK
Zvar2 (A29W) monomer (SEQ ID NO 56)
   VDAKFDKEAQEAFYEILHLPNLTEEQRNWFIQSLKDDPSVSKAILAEAKKLNDAQAPK
Zvar2 (A29I) monomer (SEQ ID NO 57)
   VDAKFDKEAQEAFYEILHLPNLTEEQRNIFIQSLKDDPSVSKAILAEAKKLNDAQAPK
Zvar2 (A29M) monomer (SEQ ID NO 58)
   VDAKFDKEAQEAFYEILHLPNLTEEQRNMFIQSLKDDPSVSKAILAEAKKLNDAQAPK
Zvar2 (A29V) monomer (SEQ ID NO 59)
   VDAKFDKEAQEAFYEILHLPNLTEEQRNVFIQSLKDDPSVSKAILAEAKKLNDAQAPK
Zvar2 (A29D) monomer (SEQ ID NO 60)
   VDAKFDKEAQEAFYEILHLPNLTEEQRNDFIQSLKDDPSVSKAILAEAKKLNDAQAPK
Zvar2 (A29E) monomer (SEQ ID NO 61)
   VDAKFDKEAQEAFYEILHLPNLTEEQRNEFIQSLKDDPSVSKAILAEAKKLNDAQAPK
Zvar2 (A29H) monomer (SEQ ID NO 62)
   VDAKFDKEAQEAFYEILHILPNLTEEQRNHFIQSLKDDPSVSKAILAEAKKLNDAQAPK
Zvar2 (A29R) monomer (SEQ ID NO 63)
   VDAKFDKEAQEAFYEILHLPNLTEEQRNRFIQSLKDDPSVSKAILAEAKKLNDAQAPK
Zvar2 (A29K) monomer (SEQ ID NO 64)
   VDAKFDKEAQEAFYEILHLPNLTEEQRNKFIQSLKDDPSVSKAILAEAKKLNDAQAPK
Zvar2 (Δ235,236,237) dimer (SEQ ID NO 65) 
VDAKFDKEAQEAFYEILHLPNLTEEQRNAFIQSLKDDPSVSKAILAEAKKLNDAQAPKV
FDKEAQEAFYEILHLPNLTEEQRNAFIQSLKDDPSVSKAILAEAKKLNDAQAPK
Zvar2 (Δ 233,234,235) dimer (SEQ ID NO 66) 
VDAKFDKEAQEAFYEILHLPNLTEEQRNAFIQSLKDDPSVSKAILAEAKKLNDAQAPAK
FDKEAQEAFYEILHLPNLTEEQRNAFIQSLKDDPSVSKAILAEAKKLNDAQAPK
Zvar2 (Δ 5-1) dimer (SEQ ID NO 67) 
VDAKFDKEAQEAFYEILHLPNLTEEQRNAFIQSLKDDPSVSKAILAEAKKLNDAQAKFD
KEAQEAFYEILHLPNLTEEQRNAFIQSLKDDPSVSKAILAEAKKLNDAQAPK
Zvar2 (Δ 5-2) dimer (SEQ ID NO 68) 
VDAKFDKEAQEAFYEILHLPNLTEEQRNAFIQSLKDDPSVSKAILAEAKKLNDAQAPKV
DAAQEAFYEILHLPNLTEEQRNAFIQSLKDDPSVSKAILAEAKKLNDAQAPK
Zvar2 (D3C-term) dimer (SEQ ID NO 69) 
VDAKFDKEAQEAFYEILHLPNLTEEQRNAFIQSLKDDPSVSKAILAEAKKLNDAQVDAK
FDKEAQEAFYEILHLPNLTEEQRNAFIQSLKDDPSVSKAILAEAKKLNDAQAPK
Zvar2 (D3N-term) dimer (SEQ ID NO 70) 
VDAKFDKEAQEAFYEILHLPNLTEEQRNAFIQSLKDDPSVSKAILAEAKKLNDAQAPKK
FDKEAQEAFYEILHLPNLTEEQRNAFIQSLKDDPSVSKAILAEAKKLNDAQAPK
Zvar2 (D8N-term) dimer (SEQ ID NO 71) 
VDAKFDKEAQEAFYEILHLPNLTEEQRNAFIQSLKDDPSVSKAILAEAKKLNDAQAPKA
QEAFYEILHLPNLTEEQRNAFIQSLKDDPSVSKAILAEAKKLNDAQAPK
Zvar2 (Linker+8) dimer (SEQ ID NO 72) 
VDAKFDKEAQEAFYEILHLPNLTEEQRNAFIQSLKDDPSVSKAILAEAKKLNDAQAPKY
EDGKQYTVDAKFDKEAQEAFYEILHLPNLTEEQRNAFIQSLKDDPSVSKAILAEAKKLN
DAQAPK
Zvar2 (Linker+4) dimer (SEQ ID NO 73) 
VDAKFDKEAQEAFYEILHLPNLTEEQRNAFIQSLKDDPSVSKAILAEAKKLNDAQAPKY
EDGVDAKFDKEAQEAFYEILHLPNLTEEQRNAFIQSLKDDPSVSKAILAEAKKLNDAQA
PK
Zvar2 (Δ Q9) monomer (SEQ ID NO 74)
   VDAKFDKEQEAFYEILHLPNLTEEQRNAFIQSLKDDPSVSKAILAEAKKLNDAQAPK
Zvar2 (Δ Q40) monomer (SEQ ID NO 75)
   VDAKFDKEAQEAFYEILHLPNLTEEQRNAFIQSLKDDPSSKAILAEAKKLNDAQAPK
Zvar2 (Δ A42) monomer (SEQ ID NO 76)
   VDAKFDKEAQEAFYEILHLPNLTEEQRNAFIQSLKDDPSVSAILAEAKKLNDAQAPK
Zvar2 (Δ N43) monomer (SEQ ID NO 77)
   VDAKFDKEAQEAFYEILHLPNLTEEQRNAFIQSLKDDPSVSKILAEAKKLNDAQAPK
Zvar2 (Δ L44) monomer (SEQ ID NO 78)
   VDAKFDKEAQEAFYEILHLPNLTEEQRNAFIQSLKDDPSVSKALAEAKKLNDAQAPK
Zvar2 (E11N,A12F) monomer (SEQ ID NO 79)
   VDAKFDKEAQNFFYEILHLPNLTEEQRNAFIQSLKDDPSVSKAILAEAKKLNDAQAPK
Zvar2 (E11N,A12Y) monomer (SEQ ID NO 80)
   VDAKFDKEAQNYFYEILHLPNLTEEQRNAFIQSLKDDPSVSKAILAEAKKLNDAQAPK
Zvar2 (E11N,A12K) monomer (SEQ ID NO 81)
   VDAKFDKEAQNKFYEILHLPNLTEEQRNAFIQSLKDDPSVSKAILAEAKKLNDAQAPK
Zvar2 (E11N,A12R) monomer (SEQ ID NO 82)
   VDAKFDKEAQNRFYEILBLPNLTEEQRNAFIQSLKDDPSVSKAILAEAKKLNDAQAPK
Zvar2 (L22F) monomer (SEQ ID NO 83)
   VDAKFDKEAQEAFYEILHLPNFTEEQRNAFIQSLKDDPSVSKAILAEAKKLNDAQAPK
Zvar2 (A43N,I44F) monomer (SEQ ID NO 84)
   VDAKFDKEAQEAFYEILHLPNLTEEQRNAFIQSLKDDPSVSKNFLAEAKKLNDAQAPK
Zvar2 (A43N,I44Y) monomer (SEQ ID NO 85)
   VDAKFDKEAQEAFYEILHLPNLTEEQRNAFIQSLKDDPSVSKNYLAEAKKLNDAQAPK
Zvar2 (A43N,I44W) monomer (SEQ ID NO 86)
   VDAKFDKEAQEAFYEILHLPNLTEEQRNAFIQSLKDDPSVSKNWLAEAKKLNDAQAPK
Zvar2 (A43N,I44R) monomer (SEQ ID NO 87)
   VDAKFDKEAQEAFYEILHLPNLTEEQRNAFIQSLKDDPSVSKNRLAEAKKLNDAQAPK
Zvar2 (A43N,I44K) monomer (SEQ ID NO 88)
   VDAKFDKEAQEAFYEILHLPNLTEEQRNAFIQSLKDDPSVSKNKLAEAKKLNDAQAPK
Zvar2 (D53F) monomer (SEQ ID NO 89)
   VDAKFDKEAQEAFYEILHLPNLTEEQRNAFIQSLKDDPSVSKAILAEAKKLNFAQAPK
Zvar2 (D53Y) monomer (SEQ ID NO 90)
   VDAKFDKEAQEAFYEILHLPNLTEEQRNAFIQSLKDDPSVSKAILAEAKKLNYAQAPK
Zvar2 (D53W) monomer (SEQ ID NO 91)
   VDAKFDKEAQEAFYEILHLPNLTEEQRNAFIQSLKDDPSVSKAILAEAKKLNWAQAPK
Zvar2 (D53K) monomer (SEQ ID NO 92)
   VDAKFDKEAQEAFYEILHLPNLTEEORNAFIOSLKDDPSVSKAILAEAKKLNKAOAPK
Zvar2 (D53R) monomer (SEQ ID NO 93)
   VDAKFDKEAOEAFYEILHLPNLTEEQRNAFIOSLKDDPSVSKAILAEAKKLNRAQAPK

## Claims

1. A recombinant protein comprising an Fc-binding polypeptide and, linked to the N-terminus of said Fc-binding polypeptide, an N-terminal spacer having a length such that the number of amino acid residues between a signal peptide cleaving site and an N-terminus proximal alpha-helix of said Fc-binding polypeptide is 14-24, and wherein the two N-terminal amino acids of said N-terminal spacer are alanine followed by glutamine.

2. The recombinant protein of claim 1, wherein said Fc-binding polypeptide comprises at least three alpha helices.

3. The recombinant protein of any preceding claim, wherein at most two amino acid residues in said N-terminal spacer are selected from the group consisting of lysine and arginine, and/or wherein said N-terminal spacer consists of 8-24 amino acid residues.

4. The recombinant protein of any preceding claim, wherein said N-terminal spacer has at least 80% sequence identity to, or is defined by, an amino acid sequence selected from the group consisting of SEQ ID NO. 16-18, 29-30, 33-40, 43-45 and 47.

5. The recombinant protein of any preceding claim, wherein said Fc-binding polypeptide comprises one or more Fc-binding domains derived from *Staphylococcus aureus* Protein A.

6. The recombinant protein of claim 5, wherein said Fc-binding domains are alkali-stabilized Fc-binding domains, optionally having at least 90% sequence identity to an amino acid sequence selected from the group consisting of SEQ ID NO. 1-11 and 48-64.

7. The recombinant protein of any one of claims 5-6, wherein said Fc-binding polypeptide comprises a multimer of at least four Fc-binding domains.

8. The recombinant protein of any preceding claim, further comprising at, or adjacent to, the C-terminus a coupling moiety, which optionally comprises a cysteine residue and/or a plurality of lysine residues.

9. The recombinant protein of any preceding claim, wherein said N-terminal spacer is alkali stable due to the absence of asparagines, and/or consists of amino acid residues selected from the group consisting of alanine, aspartic acid, glutamine, glutamic acid, glycine, histidine, lysine, phenylalanine, serine, threonine, tryptophan, tyrosine and valine.

10. A nucleic acid molecule encoding for the recombinant protein of any preceding claim, said nucleic acid molecule comprising the following elements in the 5' to 3' direction, said elements being operatively linked:
a) an inducible or constitutive promoter DNA sequence;
b) a DNA sequence encoding a signal peptide;
c) a DNA sequence encoding an N-terminal spacer; and
d) a DNA sequence encoding an Fc-bindingpolypeptide.

11. A cloning vector which expresses and secretes the recombinant protein of any preceding claim into the bacterial periplasm of a gram-negative cell, said cloning vector comprising the nucleic acid molecule of claim 10.

12. A gram-negative bacterium comprising the cloning vector of claim 11, which bacterium is optionally identified as *Escherichia coli,* such as *Escherichia coli* K12 or *Escherichia coli* K12-017.

13. A method of expressing and secreting the recombinant protein of any preceding claim in a gram-negative bacterium, said method comprising the steps of:
i) providing the gram-negative bacterium of claim 12; and
ii) culturing said gram-negative bacterium.

14. A separation matrix comprising the recombinant protein of any one of claims 1-9, covalently linked to a support, optionally comprising porous particles, such as cross-linked polysaccharide particles.

15. The separation matrix of claim 14, wherein said recombinant protein is covalently linked to said support via a thioether bond or one or more amide bonds.

16. The separation matrix of any one of claims 14-15, wherein said separation matrix is alkali stable, such as wherein the IgG capacity of the matrix after 24 h incubation time in 0.5 M NaOH at 22 +/- 2 °C is at least 80% of the IgG capacity before the incubation.

17. A method of separating an immunoglobulin, comprising the steps of:
i) providing the separation matrix of any one of claims 14-16;
ii) contacting said separation matrix with a liquid sample containing an immunoglobulin, to bind said immunoglobulin;
iii) optionally washing said separation matrix with a washing liquid;
iv) contacting said separation matrix with an elution liquid, to elute said immunoglobulin.

18. The method of claim 17, wherein in step a) said recombinant protein comprises one or more Fc-binding domains derived from *Staphylococcus aureus* Protein A and wherein said method further comprises, after step iv), a step v) of cleaning said separation matrix with a cleaning liquid, wherein said cleaning liquid optionally comprises at least 0.1 M NaOH or KOH, such as at least 0.5 M NaOH or KOH, or 0.5-2.5 M NaOH or KOH.

## Patentansprüche

1. Rekombinantes Protein, das ein Fc-bindendes Polypeptid und einen mit dem N-Terminus des Fc-bindenden Polypeptids verbundenen N-terminalen Spacer umfasst, der eine solche Länge aufweist, dass die Anzahl von Aminosäureresten zwischen einer Signalpeptid-Spaltstelle und einer N-terminalen proximalen Alpha-Helix des Fc-bindenden Polypeptids 14-24 beträgt, und wobei die beiden N-terminalen Aminosäuren des N-terminalen Spacers Alanin gefolgt von Glutamin sind.

2. Rekombinantes Protein nach Anspruch 1, wobei das Fc-bindende Polypeptid mindestens drei Alpha-Helices umfasst.

3. Rekombinantes Protein nach einem vorstehenden Anspruch, wobei höchstens zwei Aminosäurereste in dem N-terminalen Spacer aus der Gruppe ausgewählt sind, die aus Lysin und Arginin besteht, und/oder wobei der N-terminale Spacer aus 8-24 Aminosäureresten besteht.

4. Rekombinantes Protein nach einem vorstehenden Anspruch, wobei der N-terminale Spacer mindestens 80 % Sequenzidentität mit einer Aminosäuresequenz aufweist oder durch eine solche definiert ist, die aus der Gruppe ausgewählt ist, die aus SEQ ID NR. 16-18, 29-30, 33-40, 43-45 und 47 besteht.

5. Rekombinantes Protein nach einem vorstehenden Anspruch, wobei das Fc-bindende Polypeptid eine oder mehrere Fc-Bindungsdomänen umfasst, die von Protein A aus *Staphylococcus aureus* stammen.

6. Rekombinantes Protein nach Anspruch 5, wobei die Fc-Bindungsdomänen alkalistabilisierte Fc-Bindungsdomänen sind, die optional mindestens 90 % Sequenzidentität mit einer Aminosäuresequenz aufweisen, die aus der Gruppe ausgewählt ist, die aus SEQ ID NR. 1-11 und 48-64 besteht.

7. Rekombinantes Protein nach einem der Ansprüche 5-6, wobei das Fc-bindende Polypeptid ein Multimer aus mindestens vier Fc-Bindungsdomänen umfasst.

8. Rekombinantes Protein nach einem vorstehenden Anspruch, das weiter am oder neben dem C-Terminus eine Kopplungseinheit umfasst, die optional einen Cysteinrest und/oder eine Vielzahl von Lysinresten umfasst.

9. Rekombinantes Protein nach einem vorstehenden Anspruch, wobei der N-terminale Spacer aufgrund der Abwesenheit von Asparaginen alkalistabil ist und/oder aus Aminosäureresten besteht, die aus der Gruppe ausgewählt sind, die aus Alanin, Asparaginsäure, Glutamin, Glutaminsäure, Glycin, Histidin, Lysin, Phenylalanin, Serin, Threonin, Tryptophan, Tyrosin und Valin besteht.

10. Nukleinsäuremolekül, das für das rekombinante Protein nach einem vorstehenden Anspruch codiert, wobei das Nukleinsäuremolekül die folgenden Elemente in der 5'- bis 3'-Richtung umfasst, wobei die Elemente operativ verknüpft sind:
a) eine induzierbare oder konstitutive Promotor-DNA-Sequenz;
b) eine DNA-Sequenz, die ein Signalpeptid codiert;
c) eine DNA-Sequenz, die einen N-terminalen Spacer codiert; und
d) eine DNA-Sequenz, die ein Fc-bindendes Polypeptid codiert.

11. Klonierungsvektor, der das rekombinante Protein nach einem vorstehenden Anspruch exprimiert und in das bakterielle Periplasma einer gramnegativen Zelle sezerniert, wobei der Klonierungsvektor das Nukleinsäuremolekül nach Anspruch 10 umfasst.

12. Gramnegatives Bakterium, das den Klonierungsvektor nach Anspruch 11 umfasst, wobei das Bakterium optional als *Escherichia coli* identifiziert wird, wie beispielsweise *Escherichia coli* K12 oder *Escherichia coli* K12-017.

13. Verfahren zum Exprimieren und Sezernieren des rekombinanten Proteins nach einem vorstehenden Anspruch in einem gramnegativen Bakterium, wobei das Verfahren die folgenden Schritte umfasst:
i) Bereitstellen des gramnegativen Bakteriums nach Anspruch 12; und
ii) Kultivieren des gramnegativen Bakteriums.

14. Trennmatrix, die das rekombinante Protein nach einem der Ansprüche 1-9 umfasst, das kovalent an einen Träger gebunden ist, der optional poröse Partikel, wie beispielsweise vernetzte Polysaccharidpartikel, umfasst.

15. Trennmatrix nach Anspruch 14, wobei das rekombinante Protein über eine Thioetherbindung oder eine oder mehrere Amidbindungen kovalent an den Träger gebunden ist.

16. Trennmatrix nach einem der Ansprüche 14-15, wobei die Trennmatrix alkalistabil ist, wobei beispielsweise die IgG-Kapazität der Matrix nach 24 Std. Inkubationszeit in 0,5 M NaOH bei 22 +/- 2 °C mindestens 80 % der IgG-Kapazität vor der Inkubation beträgt.

17. Verfahren zum Isolieren eines Immunglobulins, umfassend die folgenden Schritte:
i) Bereitstellen der Trennmatrix nach einem der Ansprüche 14-16;
ii) Kontaktieren der Trennmatrix mit einer flüssigen Probe, die ein Immunglobulin enthält, um das Immunglobulin zu binden;
iii) optional Waschen der Trennmatrix mit einer Waschflüssigkeit;
iv) Kontaktieren der Trennmatrix mit einer Elutionsflüssigkeit, um das Immunglobulin zu eluieren.

18. Verfahren nach Anspruch 17, wobei in Schritt a) das rekombinante Protein eine oder mehrere Fc-Bindungsdomänen umfasst, die von Protein A aus *Staphylococcus aureus* stammen, und wobei das Verfahren nach Schritt iv) weiter einen Schritt v) eines Reinigens der Trennmatrix mit einer Reinigungsflüssigkeit umfasst, wobei die Reinigungsflüssigkeit optional mindestens 0,1 M NaOH oder KOH, wie beispielsweise mindestens 0,5 M NaOH oder KOH, oder 0,5-2,5 M NaOH oder KOH umfasst.

## Revendications

1. Protéine recombinante comprenant un polypeptide se liant au Fc et, lié à l'extrémité N-terminale dudit polypeptide se liant au Fc, un espaceur N-terminal présentant une longueur telle que le nombre de résidus d'acides aminés entre un site de clivage du peptide signal et une hélice alpha proximale d'extrémité N-terminale dudit polypeptide se liant au Fc est 14-24, et dans laquelle les deux acides aminés N-terminaux dudit espaceur N-terminal sont l'alanine suivie de la glutamine.

2. Protéine recombinante selon la revendication 1, dans laquelle ledit polypeptide se liant au Fc comprend au moins trois hélices alpha.

3. Protéine recombinante selon une quelconque revendication précédente, dans laquelle au plus deux résidus d'acides aminés dans ledit espaceur N-terminal sont sélectionnés dans le groupe consistant en la lysine et l'arginine, et/ou dans laquelle ledit espaceur N-terminal consiste en 8 à 24 résidus d'acides aminés.

4. Protéine recombinante selon une quelconque revendication précédente, dans laquelle ledit espaceur N-terminal présente au moins 80 % d'identité de séquence avec, ou est défini par, une séquence d'acides aminés sélectionnée dans le groupe consistant en les SEQ ID N° : 16-18, 29-30, 33-40, 43-45 et 47.

5. Protéine recombinante selon une quelconque revendication précédente, dans laquelle ledit polypeptide se liant au Fc comprend un ou plusieurs domaines de liaison au Fc dérivés de la protéine A de *Staphylococcus aureus.*

6. Protéine recombinante selon la revendication 5, dans laquelle lesdits domaines de liaison au Fc sont des domaines de liaison au Fc stabilisés aux alcalis, présentant facultativement au moins 90 % d'identité de séquence avec une séquence d'acides aminés sélectionnée dans le groupe consistant en les SEQ ID N° : 1-11 et 48-64.

7. Protéine recombinante selon l'une quelconque des revendications 5-6, dans laquelle ledit polypeptide se liant au Fc comprend un multimère d'au moins quatre domaines de liaison au Fc.

8. Protéine recombinante selon une quelconque revendication précédente, comprenant en outre au niveau de l'extrémité C-terminale, ou à proximité de celle-ci, un fragment de couplage, qui comprend facultativement un résidu de cystéine et/ou une pluralité de résidus de lysine.

9. Protéine recombinante selon une quelconque revendication précédente, dans laquelle ledit espaceur N-terminal est stable aux alcalis en raison de l'absence d'asparagines, et/ou consiste en les résidus d'acides aminés sélectionnés dans le groupe consistant en l'alanine, l'acide aspartique, la glutamine, l'acide glutamique, la glycine, l'histidine, la lysine, la phénylalanine, la sérine, la thréonine, le tryptophane, la tyrosine et la valine.

10. Molécule d'acide nucléique codant pour la protéine recombinante selon une quelconque revendication précédente, ladite molécule d'acide nucléique comprenant les éléments suivants dans la direction 5' vers 3', lesdits éléments étant liés de manière fonctionnelle :
a) une séquence d'ADN promoteur inductible ou constitutive ;
b) une séquence d'ADN codant pour un peptide signal ;
c) une séquence d'ADN codant pour un espaceur N-terminal ; et
d) une séquence d'ADN codant pour un polypeptide se liant au Fc.

11. Vecteur de clonage qui exprime et sécrète la protéine recombinante selon une quelconque revendication précédente dans le périplasme bactérien d'une cellule Gram-négative, ledit vecteur de clonage comprenant la molécule d'acide nucléique selon la revendication 10.

12. Bactérie Gram-négative comprenant le vecteur de clonage selon la revendication 11, laquelle bactérie est facultativement identifiée comme *Escherichia coli,* telle que *Escherichia coli* K12 ou *Escherichia coli* K12-017.

13. Procédé d'expression et de sécrétion de la protéine recombinante selon une quelconque revendication précédente dans une bactérie Gram-négative, ledit procédé comprenant les étapes consistant à :
i) fournir la bactérie Gram-négative selon la revendication 12 ; et
ii) cultiver ladite bactérie Gram-négative.

14. Matrice de séparation comprenant la protéine recombinante selon l'une quelconque des revendications 1-9, liée de manière covalente à un support, comprenant facultativement des particules poreuses, telles que des particules de polysaccharides réticulées.

15. Matrice de séparation selon la revendication 14, dans laquelle ladite protéine recombinante est liée de manière covalente audit support par l'intermédiaire d'une liaison thioéther ou d'une ou plusieurs liaisons amide.

16. Matrice de séparation selon l'une quelconque des revendications 14-15, dans laquelle ladite matrice de séparation est stable aux alcalis, telle que dans laquelle la capacité en IgG de la matrice après 24 h de temps d'incubation dans NaOH 0,5 M à 22 +/-2 °C correspond à au moins 80 % de la capacité en IgG avant l'incubation.

17. Procédé permettant de séparer une immunoglobuline, comprenant les étapes consistant à :
i) fournir la matrice de séparation selon l'une quelconque des revendications 14-16 ;
ii) mettre en contact ladite matrice de séparation avec un échantillon liquide contenant une immunoglobuline, pour lier ladite immunoglobuline ;
iii) facultativement laver ladite matrice de séparation avec un liquide de lavage ;
iv) mettre en contact ladite matrice de séparation avec un liquide d'élution, pour éluer ladite immunoglobuline.

18. Procédé selon la revendication 17, dans lequel, à l'étape a), ladite protéine recombinante comprend un ou plusieurs domaines de liaison au Fc dérivés de la protéine A de *Staphylococcus aureus* et dans lequel ledit procédé comprend en outre, après l'étape iv), une étape v) consistant à nettoyer ladite matrice de séparer avec un liquide de nettoyage, dans lequel ledit liquide de nettoyage comprend facultativement au moins 0,1 M de NaOH ou KOH, tel qu'au moins 0,5 M de NaOH ou KOH, ou 0,5 à 2,5 M de NaOH ou KOH.
